# EUROPEAN PATENT APPLICATION

(11) **EP 2 253 634 A1**
(43) Date of publication of application: **24.11.2010**
(21) Application number: 09719988.9
(22) Date of filing: 10.03.2009
(51) Int. Cl.: C07D 487/22

(54) **LIQUID PORPHYRIN DERIVATIVE AND METHOD FOR PRODUCING THE SAME**

(30) Priority: 11.03.2008 JP 2008061553; 29.07.2008 JP 2008194643
(71) Applicant: Dai Nippon Printing Co., Ltd., Tokyo 162-8001 (JP)
(72) Inventor: MARUYAMA, Sumio, Tokyo 162-8001 (JP); SAITO, Wataru, Tokyo 162-8001 (JP)
(74) Representative: Hatt, Anna Louise
(86) International application number: PCT/JP2009/054490
(87) International publication number: WO 2009/113511

(57) **Abstract**

The present invention is to provide a liquid porphyrin derivative at 25 °C and at temperatures from 26 to 40 °C, and a method for producing the same. The liquid porphyrin derivative of the present invention is represented by the following formula (1): 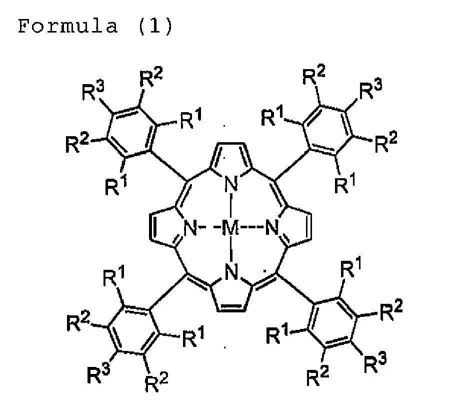 wherein M represents 2H (hydrogen atoms) or an atom or compound capable of binding covalently or coordinately to tetraphenylporphyrin; each of R¹, R², and R³ independently represents a hydrogen atom, or an alkoxy group having 7 to 14 or 15 carbon atoms represented by OR⁴; R⁴ represents a substituted or unsubstituted alkyl group having 7 to 14 or 15 carbon atoms; and all the R¹s, all the R²s, and all the R³s are respectively the same; and there are cases where the R²s and the R³s are the alkoxy groups having 7 to 14 or 15 carbon atoms represented by OR⁴, and the R¹s are hydrogen atoms, where the R¹s and the R³s are the alkoxy groups having 7 to 14 or 15 carbon atoms represented by OR⁴, and the R²s are hydrogen atoms, where the R¹s and the R²s are the alkoxy groups having 7 to 14 or 15 carbon atoms represented by OR⁴, and the R³s are hydrogen atoms, and where the R¹s, the R²s and the R³s are the alkoxy groups having 7 to 14 or 15 carbon atoms represented by OR⁴.

## Description

### Technical Field

The present invention relates to a porphyrin derivative which is in the liquid state at ordinary temperature, and a method for producing the same.

### Background Art

Porphyrin includes a macrocyclic compound comprising four pyrrole rings and four methine groups, in which each pyrrole ring and methine group are bound alternately at each α position of the pyrrole ring, and the derivative thereof, has multiple strong peaks at a visible region, and has characteristic peaks called a Q band at a red region and a Soret band around 400 nm. Since such porphyrin can coordinate metal in the center of a porphyrin skeleton and has a large n-conjugated system, it can be used as an electronic carrier, and has a sharp absorption peak in a specific wavelength region, etc. Therefore, applications to various purposes such as electronic materials, optical materials, medical materials and display materials have been attempted.

As described above, applications of the porphyrin as functional materials to a wide range of industrial fields can be expected in the future. However, there are problems that the porphyrin easily aggregates due to its rigid skeleton and the solvent solubility is poor. For example, tetraphenylporphyrin (hereinafter, it may be simply referred to as "TPP") is useful as a phosphorescent guest molecule of an organic EL element, however, the solvent solubility of TPP is significantly low. Therefore, in the case of forming a film of the organic EL element, the film needs to be formed by a vapor phase film forming method such as vacuum deposition or the like.

As an application of a porphyrin derivative, Patent Literature 1 discloses a field-effect transistor using an organic semiconductor layer containing an organic semiconductor made of a porphyrin compound having a specific structure. The porphyrin compound disclosed therein has solvent solubility and forms a film by a wet film forming method, however, it has a special structure in which a bicyclo compound is bound to a pyrrole ring.

However, even if the porphyrin has solvent solubility, it is difficult to prepare a porphyrin derivative solution having high concentration in the state that even primary particles are completely and evenly dispersed in a solvent.
On the other hand, liquid fullerene (Non Patent Literature 1) and ionic liquid at room temperature have received attention in creation of nanomaterials. For example, Bucky Gel, which can be obtained by grinding the ionic liquid and the fullerene, is known (Non Patent Literature 2).

### Citation List

[Patent Literature 1] Japanese Patent Application Laid-Open (JP-A) No. 2006-245559
[Non Patent Literature 1] J. AM. CHEM. SOC., 2006, 128, p. 10,384 to 10,385.
[Non Patent Literature 2] Science, 2003, 300, p. 2,072 to 2,074.

### Summary of Invention

### Technical Problem

If a liquid porphyrin derivative at room temperature can be obtained, a completely evenly-dispersed and highly-condensed porphyrin derivative can be obtained.
The porphyrin derivative is a compound having electron-donating ability, and is compatible with fullerene (C₆₀) and carbon nanotube having nonlinear optical property and strong electron-accepting ability. Research of the porphyrin derivative is progressing in the fields such as an organic solar battery and electron transfer reaction. Therefore, if the liquid porphyrin derivative at room temperature can be developed, there is a possibility that the liquid porphyrin derivative can exhibit specific function as a dispersion medium of the fullerene (C₆₀) and the carbon nanotube. In addition, there is also a possibility that the similar functions as the above-mentioned liquid fullerene and ionic liquid can be exhibited.

The present invention has been made in view of the above circumstances, and it is an object of the present invention to provide a porphyrin derivative which is in the liquid state at room temperature, and a method for producing the same.

### Solution to Problem

The liquid porphyrin derivative at 25 °C of the present invention is represented by the following formula (1):

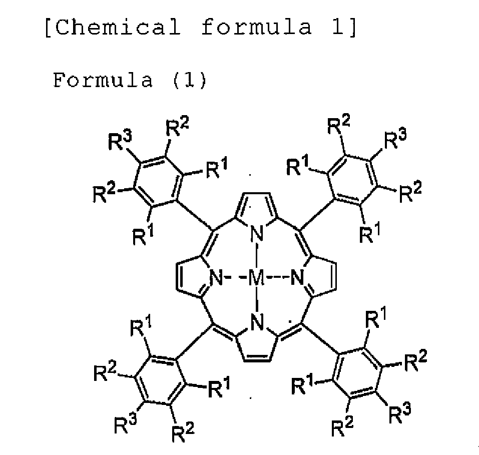

wherein M represents 2H (hydrogen atoms), or an atom or compound capable of binding covalently or coordinately to tetraphenylporphyrin; each of R¹, R², and R³ independently represents a hydrogen atom, or an alkoxy group having 7 to 14 carbon atoms represented by OR⁴; R⁴ represents a substituted or unsubstituted alkyl group having 7 to 14 carbon atoms; all the R¹s, all the R²s, and all the R³s are respectively the same; and there are cases where the R²s and the R³s are the alkoxy groups having 7 to 14 carbon atoms represented by OR⁴, and the R¹s are hydrogen atoms, where the R¹s and t h e R³s are the alkoxy groups having 7 to 14 carbon atoms represented by OR⁴, and the R²s are hydrogen atoms, where the R¹s and the R²s are the alkoxy groups having 7 to 14 carbon atoms represented by OR⁴, and the R³s are hydrogen atoms, and where the R¹s, the R²s and the R³s are the alkoxy groups having 7 to 14 carbon atoms represented by OR⁴.

While a conventional porphyrin derivative has significantly low solvent solubility, the porphyrin derivative of the present invention is in the liquid state at room temperature and has high thermal stability since the porphyrin derivative of the present invention has 3 to 5 alkoxy groups having a specific number of carbon atoms at specific positions of benzene rings as represented by the formula (1).
If the liquid porphyrin derivative of the present invention is used, a porphyrin derivative which is a pure substance, completely evenly-dispersed and highly-densed can be obtained. Therefore, a possibility of the liquid porphyrin derivative functioning as a material for creating new nanomaterials or dispersion medium can be expected.

In the formula (1) of the liquid porphyrin derivative of the present invention, it is preferable that each of the R²s and the R³s is an alkoxy group having 7 to 14 carbon atoms represented by OR⁴, and each of the R¹s is a hydrogen atom. In this case, due to the structure of the liquid porphyrin derivative, alkyl chains elongate in the direction not overlapping a surface of the porphyrin ring in the molecule of the liquid porphyrin derivative, therefore, the spacial distance between the porphyrin rings of the molecules of the porphyrin derivative is easily reduced. Thus, improvement in functionality such as electron transfer can be expected.

In the formula (1) of the liquid porphyrin derivative of the present invention, R⁴ is preferably a substituted or unsubstituted linear alkyl group for convenience of synthesis.

In addition, the present invention also provides a method for producing a liquid porphyrin derivative represented by the following formula (1):

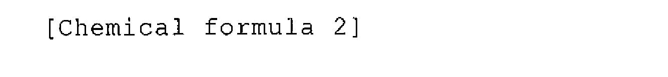

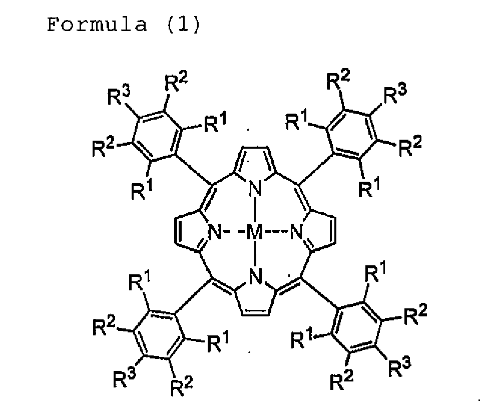

wherein M represents 2H (hydrogen atoms) or an atom or compound capable of binding covalently or coordinately to tetraphenylporphyrin; each of R¹, R², and R³ independently represents a hydrogen atom, or an alkoxy group having 7 to 14 carbon atoms represented by OR⁴; R⁴ represents a substituted or unsubstituted alkyl group having 7 to 14 carbon atoms; all the R¹s, all the R²s, and all the R³s are respectively the same; and there are cases where the R²s and the R³s are the alkoxy groups having 7 to 14 carbon atoms represented by OR⁴, and the R¹s are hydrogen atoms, where the R¹s and the R³s are the alkoxy groups having 7 to 14 carbon atoms represented by OR⁴, and the R²s are hydrogen atoms, where the R¹s and R²s are the alkoxy group having 7 to 14 carbon atoms represented by OR⁴, and the R³s are hydrogen atoms, and where the R¹s, the R²s and the R³s are the alkoxy groups having 7 to 14 carbon atoms represented by OR⁴, comprising the steps of:
reacting a benzaldehyde derivative with pyrrole, and
purifying the porphyrin derivative after reducing the unreacted benzaldehyde derivative to a benzyl alcohol derivative.

The present invention also provides a liquid porphyrin derivative at temperatures from 26 to 40 °C represented by the following formula (1):

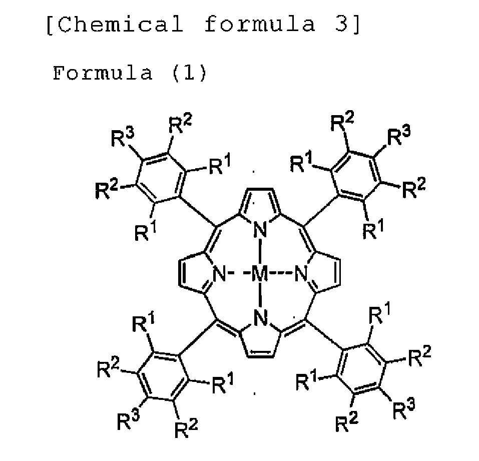

wherein M represents 2H (hydrogen atoms) or an atom or compound capable of binding covalently or coordinately to tetraphenylporphyrin; each of R¹, R², and R³ independently represents a hydrogen atom, or an alkoxy group having 15 carbon atoms represented by OR⁴; R⁴ represents a substituted or unsubstituted alkyl group having 15 carbon atoms; and all the R¹s, all the R²s, and all the R³s are respectively the same; and there are cases where the R²s and the R³s are the alkoxy groups having 15 carbon atoms represented by OR⁴, and the R¹s are hydrogen atoms, where the R¹s and the R³s are the alkoxy groups having 15 carbon atoms represented by OR⁴, and the R²s are hydrogen atoms, where the R¹s and the R²s are the alkoxy groups having 15 carbon atoms represented by OR⁴, and the R³s are hydrogen atoms, and where the R¹s, the R²s and the R³s are the alkoxy groups having 15 carbon atoms represented by OR⁴.

### Advantageous Effects of Invention

Unlike the conventional porphyrin derivative having significantly low solvent solubility, the porphyrin derivative of the present invention is a pure substance containing no solvent, and is in the liquid state at room temperature of 25 °C and at room temperatures from 26 to 40 °C while having specific characteristics of porphyrin. The porphyrin derivative of the present invention can be used as a liquid substance in a wide range of temperatures.
Accordingly, the porphyrin derivative of the present invention can be used as a dispersion medium in which various functional compounds are used as dispersoids. For example, the porphyrin derivative of the present invention is used as an electron-donating dispersion medium, a nano carbon material such as the fullerene (C₆₀) or carbon nanotube having strong electron-accepting ability is dispersed therein as a dispersoid, and the dispersion medium and the dispersoid interact under the state of high density to be able to exhibit a specific function.
In addition, by taking advantage of high electron transport property, there is a possibility that the porphyrin derivative of the present invention can be used for solar batteries, electrochemical capacitors, conductive pastes, semiconductor devices, actuators and so on.
Since the porphyrin derivative of the present invention can be used as the substance thereof without using solvents in various environments, it is possible to reduce environmental burden.
Furthermore, there is a possibility to create new nanomaterials using the porphyrin derivative of the present invention.

### Brief Description of Drawings

FIG. 1 is a photograph of an oily material (5,10,15,20-tetrakis[3,4,5-tris(undecyloxy)phenyl]porphyrin) obtained in Example 5.
FIG. 2 is a photograph of an oily material (5,10,15,20-tetrakis[3,4,5-tris(dodecyloxy)phenyl]porphyrin) obtained in Example 6.

### Description of Embodiments

Hereinafter, the liquid porphyrin derivative of the present invention and the method for producing the same will be specifically explained in order.
The liquid porphyrin derivative at 25 °C of the present invention is represented by the following formula (1):

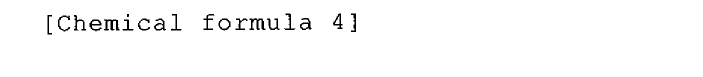

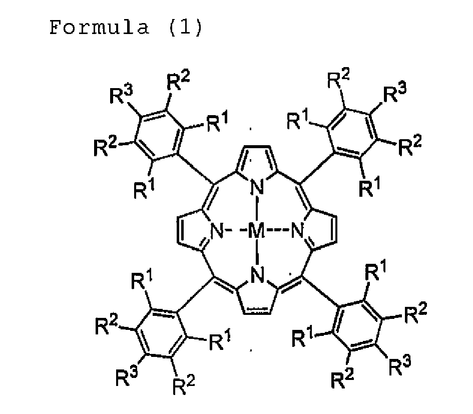

wherein M represents 2H (hydrogen atoms) or an atom or compound capable of binding covalently or coordinately to tetraphenylporphyrin; each of R¹, R², and R³ independently represents a hydrogen atom, or an alkoxy group having 7 to 14 carbon atoms represented by OR⁴; R⁴ represents a substituted or unsubstituted alkyl group having 7 to 14 carbon atoms; all the R¹s, all the R²s, and all the R³s are respectively the same; and there are cases where the R²s and the R³s are the alkoxy groups having 7 to 14 carbon atoms represented by OR⁴, and the R¹s are hydrogen atoms, where the R¹s and the R³s are the alkoxy groups having 7 to 14 carbon atoms represented by OR⁴, and the R²s are hydrogen atoms, where the R¹s and the R²s are the alkoxy groups having 7 to 14 carbon atoms represented by OR⁴, and the R³s are hydrogen atoms, and where the R¹s, the R²s and the R³s are the alkoxy groups having 7 to 14 carbon atoms represented by OR⁴.

The liquid porphyrin derivative at temperatures from 26 to 40 °C of the present invention is represented by the following formula (1):

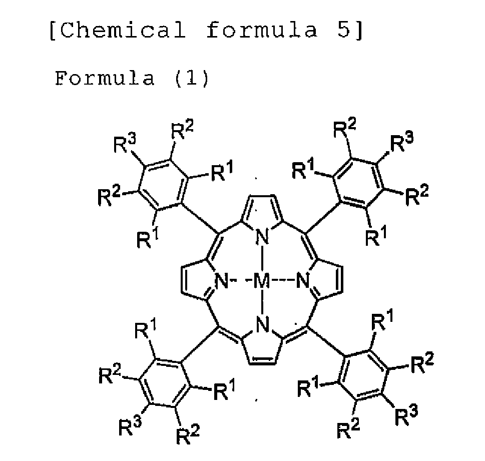

wherein M represents 2H (hydrogen atoms) or an atom or compound capable of binding covalently or coordinately to tetraphenylporphyrin; each of R¹, R², and R³ independently represents a hydrogen atom, or an alkoxy group having 15 carbon atoms represented by OR⁴; R⁴ represents a substituted or unsubstituted alkyl group having 15 carbon atoms; and all the R¹s, all the R²s, and all the R³s are respectively the same; and there are cases where the R²s and the R³s are the alkoxy groups having 15 carbon atoms represented by OR⁴, and the R¹s are hydrogen atoms, where the R¹s and the R³s are the alkoxy groups having 15 carbon atoms represented by OR⁴, and the R²s are hydrogen atoms, where the R¹s and the R²s are the alkoxy groups having 15 carbon atoms represented by OR⁴, and the R³s are hydrogen atoms, and where the R¹s, the R²s and the R³s are the alkoxy groups having 15 carbon atoms represented by OR⁴.

While the conventional porphyrin derivative has significantly low solvent solubility, the porphyrin derivative of the present invention has a specific property of being in the liquid state at room temperature, which cannot be achieved by the conventional porphyrin derivative, since the porphyrin derivative of the present invention has 3 to 5 alkoxy groups having carbon atoms of specific number at the specific positions of the benzene rings as represented by the formula (1). Herein, the liquid state at 25 °C, or at temperatures from 26 to 40 °C means the case of having fluidity at 25 °C, or at temperatures from 26 to 40 °C respectively, and the case of having a so-called paste form is included.
If the liquid porphyrin derivative of the present invention is used, a porphyrin derivative which is a pure substance, completely evenly-dispersed and highly-condensed can be obtained. Therefore, the liquid porphyrin derivative of the present invention can exhibit the function of the porphyrin derivative using the porphyrin derivative in quite a different embodiment from that of the conventional porphyrin derivative, moreover, a possibility of the liquid porphyrin derivative functioning as a dispersion medium or material for creating new nanomaterials can be expected.

The molecule of the porphyrin derivative of the present invention is designed aiming for obtaining a pure substance in the liquid state, not putting porphyrin derivatives into a liquid state by mixing them. Therefore, the porphyrin derivative of the present invention is designed to have a symmetrical molecular structure so that purification to obtain a pure substance can be easily performed. Therefore, substituents OR⁴ are substituted at 2, 4 and 6 positions, 3, 4 and 5 positions, 2, 3, 5 and 6 positions, or 2, 3, 4, 5 and 6 positions of the benzene rings, the substituents OR⁴ are the same at both 2 and 6 positions, and both 3 and 5 positions. The substitution positions and the types of the alkoxy groups of four benzene rings in the formula (1) are the same. That is, the liquid porphyrin derivative at 25 °C of the present invention is the case where the R²s and the R³s are the alkoxy groups having 7 to 14 carbon atoms represented by OR⁴, and the R¹s are hydrogen atoms, where the R¹s and the R³s are the alkoxy groups having 7 to 14 carbon atoms represented by OR⁴, and the R²s are hydrogen atoms, where the R¹s and the R²s are the alkoxy groups having 7 to 14 carbon atoms represented by OR⁴, and the R³s are hydrogen atoms, and where the R¹s, the R²s and the R³s are the alkoxy groups having 7 to 14 carbon atoms represented by OR⁴, in the formula (1). The liquid porphyrin derivative at temperatures from 26 to 40 °C is the case where the R²s and the R³s are the alkoxy groups having 15 carbon atoms represented by OR⁴, and the R¹s are hydrogen atoms, where the R¹s and the R³s are the alkoxy groups having 15 carbon atoms represented by OR⁴, and the R²s are hydrogen atoms, where the R¹s and the R²s are the alkoxy groups having 15 carbon atoms represented by OR⁴, and the R³s are hydrogen atoms, and where the R¹s, the R²s and the R³s are the alkoxy groups having 15 carbon atoms represented by OR⁴, in the formula (1).

In the formula (1) of the liquid porphyrin derivative of the present invention, it is particularly preferable that the R²s and the R³s are alkoxy groups having 7 to 14 or 15 carbon atoms represented by OR⁴, and the R¹s are hydrogen atoms. In this case, due to the structure of the liquid porphyrin derivative, alkyl chains elongate in the direction not overlapping a surface of the porphyrin ring in the molecule of the liquid porphyrin derivative, therefore, spacial distance between the porphyrin rings of the molecules of the porphyrin derivative is easily reduced. Thus, improvement in functionality such as electron transfer can be expected.

As described in Examples and Comparative examples that will be described hereinafter, if the number of carbon atoms of R⁴ is too small or too large, the melting point of the porphyrin derivative is raised, and thus the liquid state thereof maybe hardly achieved at 25 °C. Therefore, in the liquid porphyrin derivative at 25 °C of the present invention, the number of the carbon atoms of the substituent OR⁴ is selected from 7 to 14. Also, in the liquid porphyrin derivative at temperatures from 26 to 40 °C of the present invention, 15 is selected as the number of the carbon atoms of the substituent OR⁴.
In the alkoxy group having 7 to 14 or 15 carbon atoms represented by OR⁴, R⁴ represents a substituted or unsubstituted alkyl group having 7 to 14 or 15 carbon atoms. The alkyl group may include a cyclic structure besides a straight chain or a branched chain.
As the unsubstituted alkyl group having 7 to 14 or 15 carbon atoms, linear alkyl groups such as a n-heptyl group, a n-octyl group, a n-nonyl group, a n-decyl, a n-undecyl group, a n-dodecyl group, a n-tridecyl group, a n-tetradecyl group and a n-pentadecyl group can be exemplified. In addition, a branched alkyl group, in which one or more hydrogen atoms present in the chain other than the chain end of the linear alkyl group are substituted by alkyl groups such as a methyl group, an ethyl group, a n-propyl group and an isopropyl group, can be exemplified. As the branched alkyl group, a branched alkyl group, in which one or more hydrogen atoms of the linear alkyl group having 7 to 13 carbon atoms are substituted by the methyl group, the ethyl group, etc., is preferable. In the case of the branched alkyl group, for example, it is preferable to use a branched structure having symmetry. The alkyl group may be an alkyl group containing a cyclic structure such as cyclohexyl.

Moreover, examples of the substituted alkyl group having 7 to 14 or 15 carbon atoms include alkyl groups in which one or more hydrogen atoms of the alkyl group as exemplified above are substituted by substituents which do not prevent the effect of the present invention. For example, alkyl groups, in which one or more hydrogen atoms of the alkyl group are substituted by a halogen atom, a substituted or unsubstituted aromatic group, a trifluoromethyl group, a cyano group, a nitro group, an alkoxy group, an aryloxy group, an alkylthio group, dialkylamino group, etc., can be exemplified.

In the liquid porphyrin derivative of the present invention represented by the formula (1), all the R¹s, all the R²s and all the R³s are respectively the same. However, the R¹, the R² and the R³ may be the same or different from each other. From the viewpoint of synthesis of the porphyrin derivative, the case where all the substituents OR⁴ are the same is preferable.

By appropriately selecting the substituent OR⁴, the substitution number and the substitution position, various properties including viscosity and thermal property such as thermal decomposition temperature of the porphyrin derivative of the present invention can be adjusted.
Therefore, the substituent OR⁴, the substitution number and the substitution position can be appropriately selected depending on the field to which the porphyrin derivative of the present invention is applied.

For example, if the substituents OR⁴ are bound to 3, 4 and 5 positions, that is, if the R²s and the R³s are represented by OR⁴, the R¹s are hydrogen atoms and the R⁴s are linear alkyl groups having 10 to 12 carbon atoms, the melting point becomes less than 0 °C. Therefore, the porphyrin derivative of the present invention can be stably used in the liquid state under low-temperature condition, and is suitable for conductive pastes, etc.

In the formula (1), M represents 2H (hydrogen atoms) or the atom or compound capable of binding covalently or coordinately to tetraphenylporphyrin. In the case that M represents 2H, M has the structure in which the hydrogen atoms are respectively bound to two nitrogen atoms. The atom or compound capable of binding covalently or coordinately to tetraphenylporphyrin is appropriately selected from the viewpoint of controlling the functionality of the porphyrin derivative of the present invention. Examples of the atom or compound capable of binding covalently or coordinately to tetraphenylporphyrin include metallic atoms such as Ti, Co, Ni, Cu, Al, V, In, Cr, Ga, Ge, Mg, Pt, Pd, Fe and Zn, the oxides, halides such as fluoride, chloride, bromide and iodide, and hydroxides thereof. More specifically, Ti=O, AlCl, V=O, InCl, GaCl and GaOH can be exemplified.
As described in Japanese patent application No. 2007-207987 by the inventors of the present invention, thermal property such as the melting point of the porphyrin derivative is less affected by the types of the atom or compound in the porphyrin ring, and is strongly affected by the types of the substituent.

The liquid porphyrin derivative of the present invention is in the liquid state from room temperature to thermal decomposition temperature. The melting point and the freezing point of the porphyrin derivative of the present invention can be less than 0 °C depending on the types of the substituent, thus, some of the porphyrin derivative of the present invention can be in the liquid state over a wide range from less than 0 °C to thermal decomposition temperature.
The liquid porphyrin derivative of the present invention normally has a thermal decomposition temperature of 200 °C or more, and high thermal stability. Thermal property such as the thermal decomposition temperature varies depending on the selection of the substituents in the formula (1). Depending on the types of the substituents, the thermal decomposition temperature of the porphyrin derivative of the present invention is higher than that of the conventional tetraphenylporphyrin, and the thermal stability becomes high. As described above, the liquid porphyrin derivative of the present invention has high thermal decomposition temperature, therefore, it is applicable to a device whose temperature may become high when in use, a dispersion medium, and a material for creating new nanomaterials.

The method for producing the liquid porphyrin derivative of the present invention represented by the formula (1) comprises the steps of:
reacting a benzaldehyde derivative with pyrrole, and
purifying the porphyrin derivative after reducing the unreacted benzaldehyde derivative to a benzyl alcohol derivative.
As the step of reacting the benzaldehyde derivative with the pyrrole, generally, using an acid catalyst such as trifluoroacetic acid, the pyrrole is reacted with the aldehyde represented by the following formula (2):

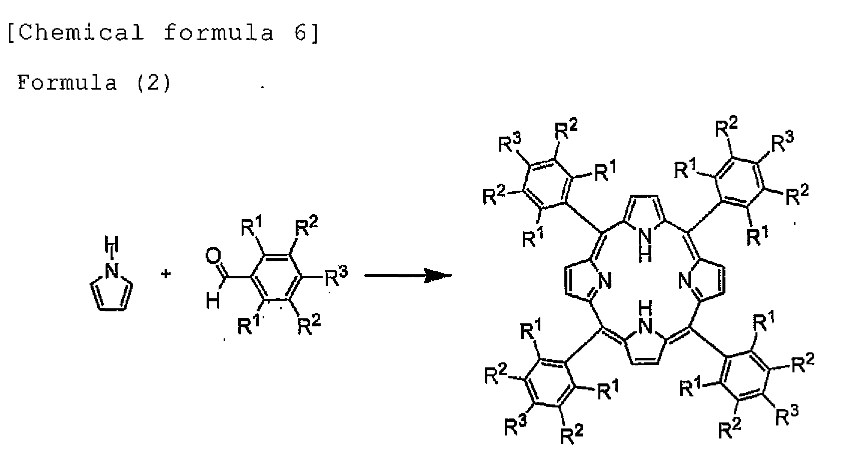

wherein each of R¹, R², and R³ independently represents a hydrogen atom, or an alkoxy group having 7 to 14 carbon atoms represented by OR⁴; R⁴ represents a substituted or unsubstituted alkyl group having 7 to 14 carbon atoms; all the R¹s, all the R²s, and all the R³s are respectively the same; and there are cases where the R²s and the R³s are the alkoxy groups having 7 to 14 carbon atoms represented by OR⁴, and the R¹s are hydrogen atoms, where the R¹s and the R³s are the alkoxy groups having 7 to 14 carbon atoms represented by OR⁴, and the R²s are hydrogen atoms, where the R¹s and R²s are the alkoxy group having 7 to 14 carbon atoms represented by OR⁴, and the R³s are hydrogen atoms, and where the R¹s, the R²s and the R³s are the alkoxy groups having 7 to 14 carbon atoms represented by OR⁴.

As the acid catalyst which causes the reaction of the pyrrole and the aldehyde represented by the formula (2), trifluoroacetic acid is preferably used. In the case of using the trifluoroacetic acid, the cyclization reaction of the pyrrole and the aldehyde represented by the formula (2) to produce porphyrin can be obtained in good yield.

The present invention comprises the step of purifying the porphyrin derivative after reducing the unreacted benzaldehyde derivative to a benzyl alcohol derivative.
It is not possible to use recrystallization for the purification of the liquid porphyrin derivative at 25 °C, because the porphyrin derivative of the present invention is in the liquid state at 25 °C. In many cases, the Rf value of the unreacted aldehyde being a material and the Rf value of the porphyrin derivative being a desired compound are almost equal, therefore, the purification of the liquid porphyrin derivative is hardly performed. Separation and purification may be performed by preparative GPC, however, there is a possibility that a substance like a porphyrin derivative is generally adsorbed on the surface of a column. Therefore, the present invention focuses attention on a substituent of the unreacted material required to be eliminated, and the unreacted aldehyde is converted to a benzyl alcohol derivative. Thus, it becomes possible to perform the separation and purification of the liquid porphyrin derivative.

The production method of the present invention is performed, for example, as described below.
Firstly, ethyl 3,4,5-trihydroxybenzoate (1 equivalent) and potassium carbonate (3.5 equivalent) are agitated in N,N-dimethylformamide (DMF) in the presence of alkyl bromide (3.5 to 4 equivalent) under an argon atmosphere at temperatures from 90 to 100 °C. After the reacted product is extracted into an organic phase using chloroform-water, the organic phase is washed twice by water, further washed by saturated sodium thiosulfate aqueous solution, and dried by anhydrous magnesium sulfate. After the organic phase is filtrated, the filtrate is condensed. Thus obtained residue is purified by Silica Gel Column Chromatography (developing solvent: hexane → chloroform), thus, ethyl 3,4,5-tris(alkoxy)benzoate is obtained.

Next, the above-obtained ethyl 3,4,5-tris(alkoxy)benzoate (1 equivalent) and potassium hydroxide (1.5 equivalent) are refluxed under heating in tetrahydrofuran(THF)-methanol-water under an argon atmosphere for several hours. After cooling the reaction solution, water is added therein, and hydrochloric acid is added therein to hydrolyze, while cooling the reaction solution with ice, and further, pH thereof is controlled to about 1. After condensing the solution followed by extracting the reacted product into an organic phase using chloroform-water, the organic phase is washed once by water, and dried by anhydrous magnesium sulfate. After the organic phase is filtrated, the filtrated is condensed. Thus obtained residue was freeze-dried, thus, 3,4,5-tris(alkoxy)benzoic acid can be obtained.

Next, while cooling a mixture of the above-obtained 3,4,5-bis(alkoxy)benzoic acid (1 equivalent) and lithium aluminum hydride (LiAlH₄) (1.2 equivalent) with ice under an argon atmosphere, THF is added therein. After the hydrogen generation stops, the mixture is refluxed overnight. After cooling the mixture to room temperature, the mixture is agitated while being cooled with ice, and ethyl acetate is added therein to consume the remaining LiAlH₄ followed by adding a small amount of water and further agitating the mixture. Next, chloroform is added therein and agitated at room temperature, and anhydrous magnesium sulfate is further added therein and agitated for several tens of minutes. After the solution is filtrated, the filtrate is condensed. Thus obtained residue is purified by Silica Gel Column Chromatography (developing solvent: chloroform → chloroform-methanol (98: 2 v/v)), thus, 3,4,5-tris(alkoxy)benzyl alcohol is obtained.

Next, a mixture of the above-obtained 3,4,5-tris(alkoxy)benzyl alcohol (1 equivalent) and manganese oxide (IV) (4 equivalent) is agitated in chloroform in the presence of anhydrous magnesium sulfate under an argon atmosphere at room temperature for several days. After the solution is filtrated, the filtrate is condensed. Thus obtained residue is separated and purified by Silica Gel Column Chromatography (developing solvent: chloroform), thus, 3,4,5-tris(alkoxy)benzaldehyde is obtained.

Next, a mixture of pyrrole (1 equivalent) and the above-obtained 3,4,5-tris(alkoxy)benzaldehyde (1 equivalent) is agitated in chloroform (about 0.01 M) at room temperature, and argon is bubbled in the solution for an hour. Subsequently, trifluoroacetic acid (TFA) (1.5 equivalent) is added therein and agitated at room temperature overnight. To thus obtained solution, 2,3-dichloro-5,6-dicyanobenzoquinone (DDQ) (1 equivalent) is added, and further agitated for 5 hours. Next, triethylamine (1.5 equivalent) is added therein and the solution is hydrolyzed followed by being condensed. Then, the obtained residue is passed through silica gel using chloroform as a developing solvent. Thus, 5,10,15,20-tetrakis[3,4,5-tris(alkoxy)phenyl]porphyrin can be obtained.
At this stage, there are many cases that the porphyrin derivative being a desired compound and the benzaldehyde derivative being a material are present in the mixture. To eliminate the unreacted material, a mixture of the porphyrin derivative and the benzaldehyde derivative being the material, and sodium borohydride (NaBF₄) are agitated in THF-ethanol (1: 1 v/v) under an argon atmosphere at room temperature overnight, followed by distilling the solvent to about half amount thereof, and the reacted product is extracted into an organic phase using chloroform-water. Then, the organic phase is washed twice by water, and dried by anhydrous sodium sulfate. After the solution is filtrated, the filtrate is condensed and thus obtained residue is purified by Silica Gel Column Chromatography (developing solvent: chloroform), and freeze-dried. Thus, the porphyrin derivative being the desired compound is obtained.
The detailed reaction conditions such as the above reaction time, reaction temperature, etc. may be appropriately adjusted, and is not particularly limited thereto.

In the case that M in the formula (1) is not the hydrogen atom, to integrate the atom or compound capable of binding covalently or coordinately to tetraphenylporphyrin, including metallic atoms such as Ti, Co, Ni, Cu, Al, V, In, Cr, Ga, Ge, Mg, Pt, Pd, Fe and Zn, the oxides, halides such as fluoride, chloride, bromide and iodide thereof, Ti=O, AlCl, V=O, InCl, GaCl, and GaOH, into the porphyrin ring, the liquid porphyrin derivative and a metallic complex such as acetylacetonato or a metallic salt to be introduced are reacted in an organic solvent at room temperature or under heating to introduce the metallic complex or salt to the porphyrin derivative.

In the above, the compound in which the alkoxy groups are substituted at 3, 4 and 5 positions is explained as an example. The compound in which the alkoxy groups are substituted at 2, 4 and 6 positions, the compound in which the alkoxy groups are substituted at 2, 3, 5 and 6 positions, or the compound in which the alkoxy groups are substituted at 2, 3, 4, 5 and 6 positions can be similarly obtained. 2,4,6-tris(alkoxy)benzaldehyde can be obtained using 2,4,6-trihydroxybenzaldehyde as a material. In addition, tetrakisalkoxybenzaldehyde and pentakisalkoxybenzaldehyde being precursors can be obtained by protecting a hydroxy group of pentafluorobenzyl alcohol or tetrafluorobenzyl alcohol by a benzyl group and reacting with sodium alkoxide to convert fluoro groups into alkoxy groups followed by deprotecting the benzyl group and oxidizing the resultant.

The liquid porphyrin derivative at temperatures from 26 to 40 °C of the present invention can be produced similarly as in the above production method. The liquid porphyrin derivative at temperatures from 26 to 40 °C of the present invention can be purified without having the step of reducing the unreacted benzaldehyde derivative to a benzyl alcohol derivative.

The liquid porphyrin derivative of the present invention obtained in the above mentioned manner has a large n-conjugated system, therefore, it can be used as an electronic carrier and is applicable to a wide range of electronics fields. By taking advantage of high electron transport property, for example, there is a possibility that the porphyrin derivative of the present invention can be used for solar batteries, electrochemical capacitors, conductive pastes, semiconductor devices, actuators and so on. In addition, there is a possibility to create new nanomaterials using the porphyrin derivative of the present invention. Furthermore, by selecting a metal, the electrical property and optical property thereof can be adjusted.
Moreover, since the liquid porphyrin derivative of the present invention has a sharp absorption peak in a specific wavelength region, etc., applications to a wide range of various purposes including electronic materials, optical materials, medical materials and display materials, such as photocatalyst, generation of photoradical, photovoltaic power, and photocurrent, using the excited state generated by an optical absorption, besides pigments.

### Examples

All reagents used in the following Examples are commercial products. Pyrrole was distilled under reduced pressure and stored under an argon atmosphere. Other reagents were not purified and used for a reaction. Silica gel 60 (product name; manufactured by MERCK) was used for the Silica Gel Column Chromatography in the purification.
In addition, JNM-LA400WB (manufactured by JEOL; 400 MHz) was used for the measurement of ¹HNMR and ¹³CNMR, and REFLEX II (product name; manufactured by BRUKER) or AXIMA-CFR plus (product name; manufactured by SHIMADZU CORPORATION) was used for the measurement of MALDI-TOF-MS.

### (Example 1)

In accordance with the following scheme, the liquid porphyrin derivative (5,10,15,20-tetrakis[3,4,5-tris(heptyloxy)phenyl]porphyrin) represented by the formula (1) was produced. The direct reduction of ethyl 3,4,5-tris(heptyloxy)benzoate to 3,4,5-tris(heptyloxy)benzyl alcohol using LiAlH₄ tends to produce low yield due to the production of by-product (it may be 1-ethyl-3,4,5-tris(heptyloxy)benzene in which only a carbonyl group of ester was reduced) or decomposition. As a result of performing a two-step reaction in which ester is hydrolyzed to be converted to carboxylic acid followed by reducing the carboxylic acid by LiAlH₄, better yield than that of the direct reduction was produced in terms of two-step processes.

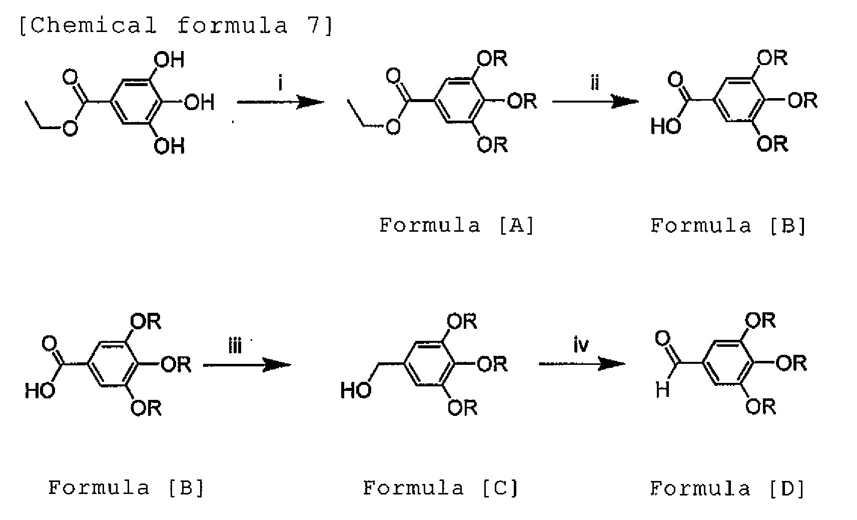

i) alkylbromide, K₂CO₃, DMF, 90 degree. ii) KOH, THF-Methanol-water, reflux. iii) LiAlH₄, THF, reflux. iv) MnO₂, CHCl₃, room temperature

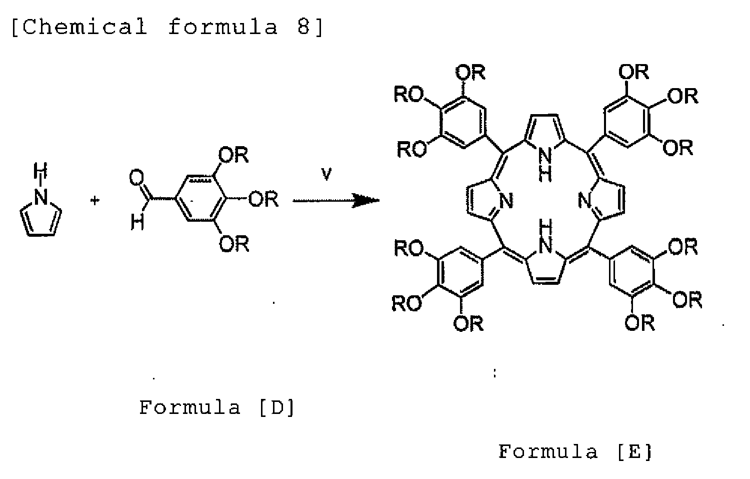

v) TFA, CHCl₃, room temperature, then DDQ following NEt₃.

### (1) Synthesis of ethyl 3,4,5-tris(heptyloxy)benzoate

Ethyl 3,4,5-trihydroxybenzoate (5.011 g, 25.286 mmol) and potassium carbonate (12.229 g, 88.484 mmol) were agitated in 40 ml of DMF in the presence of 1-bromoheptane (16.0 ml, 0.102 mol) under an argon atmosphere at temperatures from 90 to 100 °C. After the reacted product was extracted into an organic phase using chloroform-water, the organic phase was washed twice by water, further washed by saturated sodium thiosulfate solution, and dried by anhydrous magnesium sulfate. After the organic phase was filtrated, the filtrate was condensed. Thus obtained residue was purified by Silica Gel Column Chromatography (developing solvent: hexane → chloroform), thus, a desired compound (ethyl 3,4,5-tris(heptyloxy)benzoate, OR in the formula [A] is heptyloxy) (oily material, 9.006 g, yield 72.3 %) was obtained.

¹HNMR and ¹³CNMR of the obtained compound were measured and the following data was obtained.
¹H NMR (400 MHz, CDCl₃, TMS): δ (ppm) 0.87-0.91 (m, 9H, CH₃), 1.30-1.40 (m, 21H, CH₃+CH₂), 1.44-1.51 (m, 6H, CH₂), 1.71-1.85 (m, 6H, CH₂), 4.00-4.03 (m, 6H, ArOCH₂), 4.355 (q, J=7.1 Hz, 2H, CH₃CH₂O), 7.255 (s, 2H, arom. H).
¹³ C NMR (100.4 MHz, CDCl₃): δ (ppm) 14.075, 14.09, 14.39, 22.60, 22.65, 25.98, 26.02, 29.04, 29.20, 29.29, 30.30, 31.80, 31.89 (aliphatic), 60.95, 69.13, 73.46 (ether), 107.92, 125.01, 142.25, 152.77 (aromatic), 166.47 (carbonyl).

### (2) Synthesis of 3,4,5-tris(heptyloxy)benzoic acid

Ethyl 3,4,5-tris(heptyloxy)benzoate (8.291 g, 16.826 mmol) and potassium hydroxide (1.588 g, 28.304 mmol) were refluxed under heating in THF-methanol-water (100 ml-25 ml-5 ml) under an argon atmosphere for several hours. After cooling the reaction solution, water was added therein, and hydrochloric acid was added therein, while cooling the reaction solution with ice, to hydrolyze, and further, pH thereof was controlled to about 1. After condensing the solution followed by extracting the reacted product into an organic phase using chloroform-water, the organic phase was washed once by water, and dried by anhydrous magnesium sulfate. After the organic phase was filtrated, the filtrated was condensed. Thus obtained residue was freeze-dried, thus, a desired compound (ethyl 3,4,5-tris(heptyloxy)benzoate, OR in the formula [B] is heptyloxy) (solid, 7.634g, yield 97.6 %) was obtained.

¹HNMR and ¹³CNMR of the obtained compound were measured and the following data was obtained.
¹ H NMR (400 MHz, CDCl₃, TMS): δ (ppm) 0.87-0.91 (m, 9H, CH₃), 1.29-1.52 (m, 24H, CH₂), 1.72-1.86 (m, 6H, CH₂), 4.01-4.06 (m, 6H, ArOCH₂), 7.32 (s, 2H, arom. H).
¹³ C NMR (100.4 MHz, CDCl₃): δ (ppm) 14.09, 14.10, 22.61, 22.66, 25.96, 26.01, 29.04, 29.20, 29.25, 30.31, 31.80, 31.895 (aliphatic), 69.14, 73.54 (ether), 108.465, 123.55, 143.07, 152.825 (aromatic), 171.50 (carbonyl)

### (3) Synthesis of 3,4,5-tris(heptyloxy)benzyl alcohol

While cooling a mixture of 3,4,5-bis(heptyloxy)benzoic acid (7.618 g, 16.394 mmol) and LiAlH₄ (0.754 g, 19.866 mmol) with ice under an argon atmosphere, THF (40 ml) was added therein. After the hydrogen generation stopped, the mixture was refluxed overnight. After cooling the mixture to room temperature, the mixture was agitated while being cooled with ice, and ethyl acetate was added therein to deactivate the remaining LiAlH₄ followed by adding a small amount of water and further agitating the mixture. Next, chloroform was added therein and agitated at room temperature, and anhydrous magnesium sulfate was further added therein and agitated for several minutes. After the solution was filtrated, the filtrate was condensed. Thus obtained residue was purified by Silica Gel Column Chromatography (developing solvent: chloroform→chloroform-methanol (98: 2 v/v)), thus, a desired compound (3,4,5-tris(heptyloxy)benzyl alcohol, OR in the formula [C] is heptyloxy) (solid, 6.137 g, yield 83.1 %) was obtained.

¹HNMR and ¹³CNMR of the obtained compound were measured and the following data was obtained.
¹ H NMR (400 MHz, CDCl₃, TMS): δ (ppm) 0.87-0.905 (m, 9H, CH₃), 1.25-1.50 (m, 24H, CH₂), 1.655 (t, J=6.0 Hz, 1H, OH), 1.70-1.83 (m, 6H, CH₂), 3.92-3.985 (m, 6H, ArOCH₂), 4.59 (d, J=5.6 Hz, 2H, ArCH₂OH), 6.56 (s, 2H, arom. H).
¹³ C NMR (100.4 4 MHz, CDCl₃): δ (ppm) 14.075, 14.095, 22.61, 22.66, 26.03, 26.06, 29.06, 29.25, 29.40, 30.31, 31.82, 31.915 (aliphatic), 65.67, 69.08, 73.42 (ether), 105.32, 136.00, 137.58, 153.265 (aromatic).

### (4) Synthesis of 3,4,5-tris(heptyloxy)benzaldehyde

A mixture of 3,4,5-tris(heptyloxy)benzyl alcohol (6.123 g, 13.585 mmol) and manganese oxide (IV) (4.732 g, 54.430 mmol) was agitated in chloroform in the presence of anhydrous magnesium sulfate under an argon atmosphere at room temperature for several days. After the solution was filtrated, the filtrate was condensed. Thus obtained residue was separated and purified by Silica Gel Column Chromatography (developing solvent: chloroform), thus, a desired compound (3,4,5-tris(heptyloxy)benzaldehyde, OR in the formula [D] is heptyloxy) (oily material, 4.390 g, yield 72.0 %) was obtained.

¹HNMR and ¹³CNMR of the obtained compound were measured and the following data was obtained.
¹ H NMR (400 MHz, CDCl₃, TMS): δ (ppm) 0.87-0.91 (m, 9H, CH₃), 1.28-1.40 (m, 18H, CH₂), 1.43-1.51 (m, 6H, CH₂), 1.72-1.87 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): 5 (ppm) 14.08, 14.10, 22.60, 22.65, 25.95, 26.00, 29.02, 29.18, 29.22, 30.315, 31.79, 31.88 (aliphatic), 69.18, 73.61 (ether), 107.76, 131.40, 143.755, 153.48 (aromatic), 191.34 (aldehyde).

### (5) Synthesis of 5,10,15,20-tetrakis[3,4,5-tris(heptyloxy)phenyl]porphyrin

A mixture of pyrrole (0.40 ml) and 3,4,5-tris(heptyloxy)benzaldehyde (2.077 g, 4.629 mmol) was agitated in chloroform (450 ml) at room temperature, and argon was bubbled in the solution for an hour. Subsequently, trifluoroacetic acid (TFA) (0.5 ml) was added therein and agitated at room temperature overnight. To thus obtained solution, 2,3-dichloro-5,6-dicyanobenzoquinone (DDQ) (1.082 g, 4.766 mmol) was added, and further agitated for 5 hours. Next, triethylamine (1.0 ml) was added therein and the solution was hydrolyzed followed by being condensed. Then, the obtained residue was passed through silica gel (for several times to eliminate the black component as much as possible) using chloroform as a developing solvent. At this stage, the desired compound and the benzaldehyde derivative being a material were present in the mixture at a molar number ratio of about 4: 1 (it was determined by measuring the ratio of integral by NMR). To eliminate the unreacted material, the mixture and sodium borohydride (NaBF₄) (0.207 g, 5.472 mmol) were agitated in THF-ethanol (15 ml-15ml (1: 1 v/v)) under an argon atmosphere at room temperature overnight, followed by distilling the solvent, and the reacted product was extracted into an organic phase using chloroform-water. Then, the organic phase was washed twice by water, and dried by anhydrous sodium sulfate. After the solution was filtrated, the filtrate was condensed and thus obtained residue was purified by Silica Gel Column Chromatography (developing solvent: chloroform), and freeze-dried. Thus, a desired compound (5,10,15,20-tetrakis[3,4,5-tris(heptyloxy)phenyl]porphyrin, OR in the formula [E] is heptyloxy) (red purple oily material, 0.291 g, yield 12.7 %) was obtained.

¹HNMR and MALDI-TOF-MS of the obtained compound were measured and the following data was obtained.
¹ H NMR (400 MHz, CDCl₃, TMS): δ (ppm) -2.81 (s, 2H, NH), 0.845 (t, J=6.8 Hz, 24H, CH₃), 0.96 (t, J=6.8 Hz, 12H, CH₃), 1.23-1.52 (m, 88H, CH₂), 1.62-1.70 (m, 8H, CH₂), 1.83-1.90 (m, 16H, CH₂), 1.94-2.01 (m, 8H, CH₂), 4.08 (t, J=6.6 Hz, 16H, ArOCH₂), 4.29 (t, J=6.6 Hz, 8H, ArOCH₂), 7.415 (s, 8H, arom. H), 8.94 (s, 8H, pyrrole H).
MALDI-TOF-MS (no matrix): m/z=1985.30 (M⁺).

It was not possible to use recrystallization for the purification of the porphyrin derivative which was in the liquid state because the porphyrin derivative was in the liquid state. In addition, the Rf value of the aldehyde being a material and the Rf value of the porphyrin derivative being a desired compound were almost equal. Therefore, the purification of the liquid porphyrin derivative was hardly performed. Separation and purification were considered to be performed by preparative GPC, however, there was a possibility that a substance such as a phthalocyanine derivative was generally adsorbed on the surface of the column. Therefore, the present invention focused attention on the substituent of the material, and the aldehyde was converted to the benzyl alcohol derivative. Thus, it became possible to perform the separation and purification of the liquid porphyrin derivative.

### (Example 2)

In accordance with the scheme similar to that of Example 1, 5,10,15,20-tetrakis[3,4,5-tris(octyloxy)phenyl]porphyrin was produced.

### (1) Synthesis of ethyl 3,4,5-tris(octyloxy)benzoate

A desired compound (ethyl 3,4,5-tris(octyloxy)benzoate, OR in the formula [A] is octyloxy) (oily material, 10.510 g, yield 77.8 %) was obtained similarly as in Example 1 except that ethyl 3,4,5-trihydroxybenzoate (5.003 g, 25.245 mmol), potassium carbonate (12.217 g, 88.397 mmol), and 1-bromooctane (17.6 ml, 0.101 mol) were used.

¹HNMR and ¹³CNMR of the obtained compound were measured and the following data was obtained.
¹ H NMR (400 MHz, CDCl₃, TMS): δ (ppm) 0.87-0.90 (m, 9H, CH₃), 1.24-1.40 (m, 27H, CH₃+CH₂), 1.44-1.51 (m, 6H, CH₂), 1.705-1.85 (m, 6H, CH₂), 4.00-4.03 (m, 6H, ArOCH₂), 4.35 (q, J=7.2 Hz, 2H, CH₃CH₂O), 7.25 (s, 2H, arom. H).
¹³ C NMR (100.4 MHz, CDCl₃): δ (ppm) 14.10, 14.40, 22.66, 22.685, 25.79, 26.025, 26.06, 29.13, 29.28, 29.34, 29.35, 29.50, 30.295, 31.81, 31.88 (aliphatic), 60.96, 69.11, 73.46 (ether), 107.88, 125.005, 142.21, 152.76 (aromatic), 166.47 (carbonyl)

### (2) Synthesis of 3,4,5-tris(octyloxy)benzoic acid

A desired compound (ethyl 3,4,5-tris(octyloxy)benzoate, OR in the formula [B] is octyloxy) (solid, 9.670 g, yield 97.2 %) was obtained similarly as in Example 1 except that ethyl 3,4,5-tris(octyloxy)benzoate (10.500 g, 19.633 mmol), potassium hydroxide (1.616 g, 28.803 mmol), and THF-methanol-water (100 ml-20 ml-5 ml) were used.

¹HNMR and ¹³CNMR of the obtained compound were measured and the following data was obtained.
¹ H NMR (400 MHz, CDCl₃, TMS): 5 (ppm) 0.87-0.91 (m, 9H, CH₃), 1.245-1.38 (m, 24H, CH₂), 1.43-1.52 (m, 6H, CH₂), 1.72-1.86 (m, 6H, CH₂), 4.01-4.06 (m, 6H, ArOCH₂), 7.32 (s, 2H, arom. H). ¹³ C NMR (100.4 MHz, CDCl₃): δ (ppm) 14.12, 22.68, 22.70, 26.035, 26.08, 29.27, 29.29, 29.35, 29.37, 29.51, 30.33, 31.84, 31.905, 32.77 (aliphatic), 69.17, 73.56 (ether), 108.50, 123.57, 143.10, 152.85 (aromatic), 171.45 (carbonyl).

### (3) Synthesis of 3,4,5-tris(octyloxy)benzyl alcohol

A desired compound (3,4,5-tris(octyloxy)benzyl alcohol, OR in the formula [C] is octyloxy) (solid, 8.568 g, 91.2 %) was obtained similarly as in Example 1 except that 3,4,5-bis(octyloxy)benzoic acid (9.644 g, 19.070 mmol) and LiAlH₄ (0.887 g, 23.370 mmol) were used.

¹HNMR and ¹³CNMR of the obtained compound were measured and the following data was obtained.
¹ H NMR (400 MHz, CDCl₃, TMS): δ (ppm) 0.87-0.90 (m, 9H, CH₃), 1.24-1.36 (m, 24H, CH₂), 1.43-1.50 (m, 6H, CH₂), 1.68 (t, J=6.2 Hz, 1H, OH), 1.70-1.83 (m, 6H, CH₂), 3.92-3.98 (m, 6H, ArOCH₂), 4.59 (d, J=6.0 Hz, 2H, ArCH₂OH), 6.56 (s, 2H, arom. H).
¹³ C NMR (100.4 MHz, CDCl₃): δ (ppm) 14.095, 22.66, 22.69, 25.72, 26.08, 26.10, 29.29, 29.35, 29.38, 29.55, 30.30, 31.82, 31.90, 32.79 (aliphatic), 65.67, 69.06, 73.41 (ether), 105.27, 136.00, 137.51, 153.25 (aromatic).

### (4) Synthesis of 3,4,5-tris(octyloxy)benzaldehyde

A desired compound (3,4,5-tris(octyloxy)benzaldehyde, OR in the formula [D] is octyloxy) (oily material, 7.028 g, yield 82.5 %) was obtained similarly as in Example 1 except that 3,4,5-tris(octyloxy)benzyl alcohol (8.554 g, 17.359 mmol) and manganese oxide (IV) (6.044 g, 69.522 mmol) were used.

¹HNMR and ¹³CNMR of the obtained compound were measured and the following data was obtained.
¹ H NMR (400 MHz, CDCl₃, TMS): δ (ppm) 0.87-0.905 (m, 9H, CH₃), 1.26-1.38 (m, 24H, CH₂), 1.44-1.52 (m, 6H, CH₂), 1.72-1.865 (m, 6H, CH₂), 4.03 (t, J=6.6 Hz, 4H, ArOCH₂), 4.06 (t, J=6.4 Hz, 2H, ArOCH₂), 7.08 (s, 2H, arom. H), 9.83 (s, 1H, CHO).
¹³ C NMR (100.4 MHz, CDCl₃): δ (ppm) 14.09, 22.65, 22.68, 26.00, 26.045, 29.23, 29.26, 29.32, 29.34, 29.47, 30.32, 31.80, 31.87 (aliphatic), 69.205, 73.62 (ether), 107.81, 131.41, 143.80, 153.50 (aromatic), 191.31 (aldehyde).

### (5) Synthesis of 5,10,15,20-tetrakis[3,4,5-tris(octyloxy)phenyl]porphyrin

A desired compound (5,10,15,20-tetrakis[3,4,5-tris(octyloxy)phenyl]porphyrin, OR in the formula [E] is octyloxy) (red purple oily material, 0.256 g, yield 11.4 %) was obtained similarly as in Example 1 except that pyrrole (0.35 ml), 3,4,5-tris(octyloxy)benzaldehyde (2.039 g, 4.155 mmol), chloroform (420 ml), trifluoroacetic acid(TFA) (0.455 ml), DDQ (0.928 g, 4.088 mmol), triethylamine (1.0 ml), NaBH₄ (0.326 g, 8.617 mmol), and THF-ethanol (10 ml-10 ml (1:1 v/v)) were used.

¹HNMR and MALDI-TOF-MS of the obtained compound were measured and the following data was obtained. ¹ H NMR (400 MHz, CDCl₃, TMS): δ (ppm) -2.81 (s, 2H, NH), 0.83 (t, J=6.8 Hz, 24H, CH₃), 0.935 (t, J=7.0 Hz, 12H, CH₃), 1.23-1.52 (m, 112H, CH₂), 1.63-1.70 (m, 8H, CH₂), 1.825-1.89 (m, 16H, CH₂), 1.935-2.005 (m, 8H, CH₂), 4.08 (t, J=6.4 Hz, 16H, ArOCH₂), 4.29 (t, J=6.6 Hz, 8H, ArOCH₂), 7.41 (s, 8H, arom. H), 8.94 (s, 8H, pyrrole H).
MALDI-TOF-MS (no matrix): m/z=2153.47 (M⁺).

### (Example 3)

In accordance with the scheme similar to that of Example 1, 5,10,15,20-tetrakis[3,4,5-tris(nonyloxy)phenyl]porphyrin was produced.

### (1) Synthesis of ethyl 3,4,5-tris(nonyloxy)benzoate

A desired compound (ethyl 3,4,5-tris(nonyloxy)benzoate, OR in the formula [A] is nonyloxy) (oily material, 8.185 g, yield 56.2 %) was obtained similarly as in Example 1 except that ethyl 3,4,5-trihydroxybenzoate (5.007 g, 25.266 mmol), potassium carbonate (12.232 g, 88.506 mmol), and 1-bromononane (17.0 ml, 88.958 mmol) were used.

¹HNMR and ¹³CNMR of the obtained compound were measured and the following data was obtained.
¹ H NMR (400 MHz, CDCl₃, TMS): δ (ppm) 0.865-0.90 (m, 9H, CH₃), 1.28-1.40 (m, 33H, CH₃+CH₂), 1.44-1.51 (m, 6H, CH₂), 1.705-1.85 (m, 6H, CH₂), 4.00-4.03 (m, 6H, ArOCH₂), 4.35 (q, J=7.2 Hz, 2H, CH₃CH₂O), 7.25 (s, 2H, arom. H).
¹³ C NMR (100.4 MHz, CDCl₃): δ (ppm) 14.08, 14.38, 22.66, 25.79, 26.04, 26.07, 28.49, 29.16, 29.27, 29.31, 29.35, 29.38, 29.57, 29.66, 30.31, 31.88, 31.92 (aliphatic), 60.93, 69.16, 73.46 (ether), 107.98, 125.03, 142.315, 152.78 (aromatic), 166.45 (carbonyl).

### (2) Synthesis of 3,4,5-tris(nonyloxy)benzoic acid

A desired compound (ethyl 3,4,5-tris(nonyloxy)benzoate, OR in the formula [B] is nonyloxy) (solid, 7.693 g, yield 99.2 %) was obtained similarly as in Example 1 except that ethyl 3,4,5-tris(nonyloxy)benzoate (8.148 g, 14.124 mmol), potassium hydroxide (1.183 g, 21.085 mmol), and THF-methanol-water (80 ml-20 ml-8 ml) were used.

¹HNMR and ¹³CNMR of the obtained compound were measured and the following data was obtained.
¹ H NMR (400 MHz, CDCl₃, TMS): δ (ppm) 0.87-0.91 (m, 9H, CH₃), 1.28-1.38 (m, 30H, CH₂), 1.43-1.51 (m, 6H, CH₂), 1.715-1.86 (m, 6H, CH₂), 4.01-4.06 (m, 6H, ArOCH₂), 7.32 (s, 2H, arom. H).
¹³ C NMR (100.4 MHz, CDCl₃): δ (ppm) 14.095, 22.68, 25.72, 26.03, 26.07, 29.27, 29.28, 29.36, 29.39, 29.55, 29.58, 29.665, 30.32, 31.895, 31.93, 32.76 (aliphatic), 69.19, 73.55 (ether), 108.55, 123.56, 143.16, 152.85 (aromatic), 171.45 (carbonyl).

### (3) Synthesis of 3,4,5-tris(nonyloxy)benzyl alcohol

A desired compound (3,4,5-tris(nonyloxy)benzyl alcohol, OR in the formula [C] is nonyloxy) (solid, 6.915 g, 92.8 %) was obtained similarly as in Example 1 except that 3,4,5-bis(nonyloxy)benzoic acid (7.645 g, 13.929 mmol), LiAlH₄ (0.818 g, 21.552 mmol), and 35 ml of THF were used.

¹HNMR and ¹³CNMR of the obtained compound were measured and the following data was obtained.
¹ H NMR (400 MHz, CDCl₃, TMS): δ (ppm) 0.865-0.90 (m, 9H, CH₃), 1.28-1.36 (m, 30H, CH₂), 1.42-1.50 (m, 6H, CH₂), 1.68 (t, J=5.8 Hz, 1H, OH), 1.70-1.83 (m, 6H, CH₂), 3.91-3.98 (m, 6H, ArOCH₂), 4.59 (d, J=6.0 Hz, 2H, ArCH₂OH), 6.55 (s, 2H, arom. H).
¹³ C NMR (100.4 MHz, CDCl₃): δ (ppm) 14.08, 22.66, 22.685, 26.09, 26.12, 29.24, 29.28, 29.365, 29.41, 29.59, 29.60, 29.68, 30.315, 31.89, 31.94, 32.80 (aliphatic), 65.66, 69.10, 73.42 (ether), 105.35, 136.01, 137.59, 153.27 (aromatic).

### (4) Synthesis of 3,4,5-tris(nonyloxy)benzaldehyde

A desired compound (3,4,5-tris(nonyloxy)benzaldehyde, OR in the formula [D] is nonyloxy) (oily material, 5.766 g, yield 83.9 %) was obtained similarly as in Example 1 except that 3,4,5-tris(nonyloxy)benzyl alcohol (6.900 g, 12.900 mmol), manganese oxide (IV) (4.574 g, 52.613 mmol), and 35 ml of chloroform were used.

¹HNMR and ¹³CNMR of the obtained compound were measured and the following data was obtained.
¹ H NMR (400 MHz, CDCl₃, TMS): δ (ppm) 0.87-0.90 (m, 9H, CH₃), 1.28-1.38 (m, 30H, CH₂), 1.44-1.51 (m, 6H, CH₂), 1.715-1.86 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): δ (ppm) 14.08, 14.09, 22.66, 22.68, 26.01, 26.05, 29.25, 29.27, 29.34, 29.365, 29.53, 29.56, 29.65, 30.33, 31.88, 31.92 (aliphatic), 69.23, 73.62 (ether), 107.85, 131.43, 143.85, 153.51 (aromatic), 191.28 (aldehyde).

### (5) Synthesis of 5,10,15,20-tetrakis[3,4,5-tris(nonyloxy)phenyl]porphyrin

A desired compound (5,10,15,20-tetrakis[3,4,5-tris(nonyloxy)phenyl]porphyrin, OR in the formula [E] is nonyloxy) (red purple oily material, 0.295 g, yield 10.8 %) was obtained similarly as in Example 1 except that pyrrole (0.40 ml), 3,4,5-tris(nonyloxy)benzaldehyde (2.498 g, 4.688 mmol), chloroform (500 ml), trifluoroacetic acid (TFA) (0.52 ml), DDQ (1.087 g, 4.788 mmol), triethylamine (1.5 ml), NaBH₄ (0.181 g, 4.784 mmol), and THF-ethanol (10 ml-10 ml (1: 1 v/v)) were used.

¹HNMR and MALDI-TOF-MS of the obtained compound were measured and the following data was obtained. ¹ H NMR (400 MHz, CDCl₃, TMS): δ(ppm) -2.81 (s, 2H, NH), 0.83 (t, J=6.8 Hz, 24H, CH₃), 0.92 (t, J=7.0 Hz, 12H, CH₃), 1.235-1.52 (m, 136H, CH₂), 1.62-1.70 (m, 8H, CH₂), 1.82-1.89 (m, 16H, CH₂), 1.93-2.00 (m, 8H, CH₂), 4.075 (t, J=6.6 Hz, 16H, ArOCH₂), 4.29 (t, J=6.6 Hz, 8H, ArOCH₂), 7.41 (s, 8H, arom. H), 8.94 (s, 8H, pyrrole H).
MALDI-TOF-MS (no matrix): m/z=2321.939 (M⁺).

### (Example 4)

In accordance with the scheme similar to that of Example 1, 5,10,15,20-tetrakis[3,4,5-tris(decyloxy)phenyl]porphyrin was produced.

### (1) Synthesis of ethyl 3,4,5-tris(decyloxy)benzoate

A desired compound (ethyl 3,4,5-tris(decyloxy)benzoate, OR in the formula [A] is decyloxy) (solid, 11.003 g, yield 70.3 %) was obtained similarly as in Example 1 except that ethyl 3,4,5-trihydroxybenzoate (5.013 g, 25.296 mmol), potassium carbonate (12.218 g, 88.404 mmol), and 1-bromodecane (18.3 ml, 87.801 mmol) were used.

¹HNMR and ¹³CNMR of the obtained compound were measured and the following data was obtained.
¹ H NMR (400 MHz, CDCl₃, TMS): δ (ppm) 0.865-0.90 (m, 9H, CH₃), 1.27-1.40 (m, 39H, CH₃+CH₂), 1.435-1.51 (m, 6H, CH₂), 1.70-1.85 (m, 6H, CH₂), 4.00-4.03 (m, 6H, ArOCH₂), 4.35 (q, J=7.1 Hz, 2H, CH₃CH₂O), 7.25 (s, 2H, arom. H).
¹³ C NMR (100.4 MHz, CDCl₃): δ (ppm) 14.095, 14.39, 22.67, 22.69, 26.045, 26.07, 29.27, 29.31, 29.34, 29.39, 29.49, 29.56, 29.575, 29.62, 29.66, 29.715, 30.31, 31.90, 31.93 (aliphatic), 60.94, 69.17, 73.475 (ether), 107.99, 125.03, 142.31, 152.78 (aromatic), 166.47 (carbonyl).

### (2) Synthesis of 3,4,5-tris(decyloxy)benzoic acid

A desired compound (ethyl 3,4,5-tris(decyloxy)benzoate, OR in the formula [B] is decyloxy) (solid, 10.320 g, yield 98.4 %) was obtained similarly as in Example 1 except that ethyl 3,4,5-tris(decyloxy)benzoate (10.990 g, 17.755 mmol), potassium hydroxide (1.565 g, 27.894 mmol), and THF-methanol-water (100 ml-25 ml-10 ml) were used.

¹HNMR and ¹³CNMR of the obtained compound were measured and the following data was obtained.
¹ H NMR (400 MHz, CDCl₃, TMS): δ (ppm) 0.865-0.90 (m, 9H, CH₃), 1.27-1.38 (m, 36H, CH₂), 1.43-1.51 (m, 6H, CH₂), 1.71-1.86 (m, 6H, CH₂), 4.01-4.06 (m, 6H, ArOCH₂), 7.32 (s, 2H, arom. H).
¹³ C NMR (100.4 MHz, CDCl₃): δ (ppm) 14.10, 22.68, 22.69, 25.72, 26.03, 26.07, 29.27, 29.34, 29.39, 29.55, 29.58, 29.62, 29.66, 29.72, 30.32, 31.91, 31.93, 32.77 (aliphatic), 69.19, 73.55 (ether), 108.555, 123.54, 143.15, 152.85 (aromatic), 171.21 (carbonyl).

### (3) Synthesis of 3,4,5-tris(decyloxy)benzyl alcohol

A desired compound (3,4,5-tris(decyloxy)benzyl alcohol, OR in the formula [C] is decyloxy) (solid, 9.242 g, yield 91.9 %) was obtained similarly as in Example 1 except that 3,4,5-bis(decyloxy)benzoic acid (10.303 g, 17.435 mmol) and LiAlH₄ (1.005 g, 26.479 mmol) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.27-1.38 (m, 36H, CH₂), 1.42-1.50 (m, 6H, CH₂), 1.63 (t, J=6.0 Hz, 1H, OH), 1.70-1.83 (m, 6H, CH₂), 3.92-3.99 (m, 6H, ArOCH₂), 4.59 (d, J=6.4 Hz, 2H, ArCH₂OH), 6.56 (s, 2H, arom. H).
¹³ C NMR (100.4 MHz, CDCl₃): δ (ppm) 14.10, 22.68, 22.69, 25.73, 26.09, 26.13, 29.31, 29.34, 29.41, 29.55, 29.59, 29.61, 29.64, 29.67, 29.74, 30.32, 31.91, 31.94 (aliphatic), 65.69, 69.11, 73.425 (ether), 105.37, 136.00, 137.61, 153.29 (aromatic).

### (4) Synthesis of 3,4,5-tris(decyloxy)benzaldehyde

A desired compound (3,4,5-tris(decyloxy)benzaldehyde, OR in the formula [D] is decyloxy) (solid, 7.323 g, yield 79.7 %) was obtained similarly as in Example 1 except that 3,4,5-tris(decyloxy)benzyl alcohol (9.217 g, 15.976 mmol) and manganese oxide (IV) (5.597 g, 64.380 mmol) were used.

¹HNMR and ¹³CNMR of the obtained compound were measured and the following data was obtained.
¹ H NMR (400 MHz, CDCl₃, TMS): δ (ppm) 0.865-0.90 (m, 9H, CH₃), 1.27-1.375 (m, 36H, CH₂), 1.44-1.51 (m, 6H, CH₂), 1.715-1.86 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): δ (ppm) 14.10, 22.68, 22.69, 26.02, 26.06, 29.25, 29.34, 29.37, 29.38, 29.53, 29.57, 29.61, 29.62, 29.65, 29.71, 30.34, 31.90, 31.93 (aliphatic), 69.23, 73.63 (ether), 107.85, 131.43, 143.85, 153.52 (aromatic), 191.29 (aldehyde).

### (5) Synthesis of 5,10,15,20-tetrakis[3,4,5-tris(decyloxy)phenyl]porphyrin

A desired compound (5,10,15,20-tetrakis[3,4,5-tris(decyloxy)phenyl]porphyrin, OR in the formula [E] is decyloxy) (red purple oily material, 0.264 g, yield 9.7 %) was obtained similarly as in Example 1 except that pyrrole (0.35 ml), 3,4,5-tris(decyloxy)benzaldehyde (2.504 g, 4.353 mmol), chloroform (450 ml), trifluoroacetic acid (TFA) (0.5 ml), DDQ (1.019 g, 4.489 mmol), triethylamine (1.0 ml), NaBH₄ (0.187 g, 4.943 mmol), and THF-ethanol (10 ml-10 ml (1: 1 v/v)) were used.

¹HNMR and MALDI-TOF-MS of the obtained compound were measured and the following data was obtained.
¹ H NMR (400 MHz, CDCl₃, TMS): δ (ppm) -2.81 (s, 2H, NH), 0.83 (t, J=7.0 Hz, 24H, CH₃), 0.91 (t, J=6.8 Hz, 12H, CH₃), 1.22-1.52 (m, 160H, CH₂), 1.63-1.70 (m, 8H, CH₂), 1.82-1.89 (m, 16H, CH₂), 1.93-2.00 (m, 8H, CH₂), 4.07 (t, J=6.2 Hz, 16H, ArOCH₂), 4.29 (t, J=6.6 Hz, 8H, ArOCH₂), 7.41 (s, 8H, arom. H), 8.94 (s, 8H, pyrrole H).
MALDI-TOF-MS (no matrix): m/z=2489.244 (M⁺).

### (Example 5)

In accordance with the scheme similar to that of Example 1, 5,10,15,20-tetrakis[3,4,5-tris(undecyloxy)phenyl]porphyrin was produced.

### (1) Synthesis of ethyl 3,4,5-tris(undecyloxy)benzoate

A desired compound (ethyl 3,4,5-tris(undecyloxy)benzoate, OR in the formula [A] is undecyloxy) (solid, 11.157 g, yield 66.4 %) was obtained similarly as in Example 1 except that ethyl 3,4,5-trihydroxybenzoate (5.036 g, 25.412 mmol), potassium carbonate (12.226 g, 88.462 mmol), and 1-bromoundecane (20.0 ml, 89.283 mmol) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.265-1.40 (m, 45H, CH₃+CH₂), 1.435-1.51 (m, 6H, CH₂), 1.70-1.845 (m, 6H, CH₂), 3.995-4.03 (m, 6H, ArOCH₂), 4.35 (q, J=7.2 Hz, 2H, CH₃CH₂O), 7.25 (s, 2H, arom. H).
¹³ C NMR (100.4 MHz, CDCl₃): δ (ppm) 14.095, 14.10, 14.40, 22.68, 22.69, 25.81, 26.05, 26.08, 29.32, 29.35, 29.39, 29.54, 29.56, 29.63, 29.64, 29.69, 29.72, 30.315, 30.32, 31.915, 31.94 (aliphatic), 60.94, 69.18, 73.475 (ether), 107.99, 125.03, 142.32, 152.79 (aromatic), 166.47 (carbonyl).

### (2) Synthesis of 3,4,5-tris(undecyloxy)benzoic acid

A desired compound (ethyl 3,4,5-tris(undecyloxy)benzoate, OR in the formula [B] is undecyloxy) (solid, 10.592 g, yield 99.4 %) was obtained similarly as in Example 1 except that ethyl 3,4,5-tris(undecyloxy)benzoate (11.129 g, 16.835 mmol), potassium hydroxide (1.513 g, 26.967 mmol), and THF-methanol-water (100 ml-25 ml-10 ml) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.27-1.37 (m, 42H, CH₂), 1.43-1.51 (m, 6H, CH₂), 1.71-1.88 (m, 6H, CH₂), 4.01-4.06 (m, 6H, ArOCH₂), 7.32 (s, 2H, arom. H).
¹³ C NMR (100.4 MHz, CDCl₃): δ (ppm) 14.095, 14.10, 22.68, 22.685, 26.03, 26.07, 29.27, 29.35, 29.38, 29.42, 29.55, 29.60, 29.62, 29.64, 29.69, 29.715, 30.32, 31.91, 31.94, 32.77 (aliphatic), 69.18, 73.54 (ether), 108.55, 123.60, 143.13, 152.85 (aromatic), 171.31 (carbonyl).

### (3) Synthesis of 3,4,5-tris(undecyloxy)benzyl alcohol

A desired compound (3,4,5-tris(undecyloxy)benzyl alcohol, OR in the formula [C] is undecyloxy) (solid, 10.061 g, yield 97.3 %) was obtained similarly as in Example 1 except that 3,4,5-bis(undecyloxy)benzoic acid (10.575 g, 16.706 mmol), LiAlH₄ (0.971 g, 25.584 mmol), and THF (45 ml) were used.

¹HNMR and ¹³CNMR of the obtained compound were measured and the following data was obtained. ¹ H NMR (400 MHz, CDCl₃, TMS): δ (ppm) 0.86-0.90 (m, 9H, CH₃), 1.265-1.36 (m, 42H, CH₂), 1.42-1.50 (m, 6H, CH₂), 1.675 (t, J=6.0 Hz, 1H, OH), 1.70-1.83 (m, 6H, CH₂), 3.91-3.98 (m, 6H, ArOCH₂), 4.59 (d, J=6.0 Hz, 2H, ArCH₂OH), 6.55 (s, 2H, arom. H).
¹³ C NMR (100.4 MHz, CDCl₃): δ (ppm) 14.09, 14.095, 22.58, 22.69, 26.095, 26.13, 29.32, 29.35, 29.38, 29.41, 29.59, 29.61, 29.64, 29.73, 29.74, 30.32, 30.325, 31.91, 31.94 (aliphatic), 65.66, 69.11, 73.42 (ether), 105.36, 136.01, 137.61, 153.28 (aromatic).

### (4) Synthesis of 3,4,5-tris(undecyloxy)benzaldehyde

A desired compound (3,4,5-tris(undecyloxy)benzaldehyde, OR in the formula [D] is undecyloxy) (solid, 8.643 g, yield 86.3 %) was obtained similarly as in Example 1 except that 3,4,5-tris(undecyloxy)benzyl alcohol (10.050 g, 16.235 mmol) and manganese oxide (IV) (5.723 g, 65.829 mmol) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.27-1.375 (m, 42H, CH₂), 1.44-1.51 (m, 6H, CH₂), 1.715-1.86 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): δ (ppm) 14.10, 14.12, 22.68, 22.69, 26.02, 26.06, 29.25, 29.35, 29.37, 29.38, 29.53, 29.62, 29.63, 29.69, 29.71, 30.33, 30.34, 31.91, 31.93 (aliphatic), 69.225, 73.62 (ether), 107.84, 131.43, 143.85, 153.51 (aromatic), 191.29 (aldehyde).

### (5) Synthesis of 5,10,15,20-tetrakis[3,4,5-tris(undecyloxy)phenyl]porphyrin

A desired compound (5,10,15,20-tetrakis[3,4,5-tris(undecyloxy)phenyl]porphyrin, OR in the formula [E] is undecyloxy) (red purple oily material, 0.316 g, yield 11.4 %) was obtained similarly as in Example 1 except that pyrrole (0.30 ml), 3,4,5-tris(undecyloxy)benzaldehyde (2.564 g, 4.156 mmol), chloroform (430 ml), trifluoroacetic acid(TFA) (0.46 ml), DDQ (0.920 g, 4.052 mmol), triethylamine (1.0 ml), NaBH₄ (0.085 g, 2.247 mmol), and THF-ethanol (10 ml-10 ml (1: 1 v/v)) were used.

¹HNMR and MALDI-TOF-MS of the obtained compound were measured and the following data was obtained.
¹ H NMR (400 MHz, CDCl₃, TMS): δ (ppm) -2.81 (s, 2H, NH), 0.83 (t, J=7.0 Hz, 24H, CH₃), 0.90 (t, J=7.0 Hz, 12H, CH₃), 1.215-1.52 (m, 184H, CH₂), 1.63-1.70 (m, 8H, CH₂), 1.80-1.90 (m, 16H, CH₂), 1.915-2.00 (m, 8H, CH₂), 4.07 (t, J=6.4 Hz, 16H, ArOCH₂), 4.29 (t, J=6.6 Hz, 8H, ArOCH₂), 7.41 (s, 8H, arom. H), 8.94 (s, 8H, pyrrole H).
MALDI-TOF-MS (no matrix): m/z=2658.374 (M⁺).
A photograph of the obtained oily material is shown in FIG. 1.

### (Example 6)

In accordance with the scheme similar to that of Example 1, 5,10,15,20-tetrakis[3,4,5-tris(dodecyloxy)phenyl]porphyrin was produced.

### (1) Synthesis of ethyl 3,4,5-tris(dodecyloxy)benzoate

A desired compound (ethyl 3,4,5-tris(dodecyloxy)benzoate, OR in the formula [A] is dodecyloxy) (solid, 11.539 g, yield 64.8 %) was obtained similarly as in Example 1 except that ethyl 3,4,5-trihydroxybenzoate (5.018 g, 25.321 mmol), potassium carbonate (12.331 g, 89.222 mmol), and 1-bromododecane (22.0 ml, 91.801 mmol) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.26-1.40 (m, 51H, CH₃+CH₂), 1.435-1.51 (m, 6H, CH₂), 1.70-1.845 (m, 6H, CH₂), 3.995-4.03 (m, 6H, ArOCH₂), 4.35 (q, J=7.2 Hz, 2H, CH₃CH₂O), 7.25 (s, 2H, arom. H).
¹³ C NMR (100.4 MHz, CDCl₃): δ (ppm) 14.10, 14.40, 22.685, 25.81, 26.05, 26.08, 28.50, 29.17, 29.32, 29.36, 29.39, 29.48, 29.53, 29.57, 29.63, 29.65, 29.69, 29.715, 29.72, 29.74, 30.32, 31.92, 31.93 (aliphatic), 60.94, 69.18, 73.475 (ether), 108.00, 125.03, 142.33, 152.79 (aromatic), 166.47 (carbonyl).

### (2) Synthesis of 3,4,5-tris(dodecyloxy)benzoic acid

A desired compound (ethyl 3,4,5-tris(dodecyloxy)benzoate, OR in the formula [B] is dodecyloxy) (solid, 10.667 g, yield 97.3 %) was obtained similarly as in Example 1 except that ethyl 3,4,5-tris(dodecyloxy)benzoate (11.422 g, 16.244 mmol), potassium hydroxide (1.429 g, 25.470 mmol), and THF-methanol-water (100 ml-25 ml-10 ml) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.265-1.37 (m, 48H, CH₂), 1.43-1.51 (m, 6H, CH₂), 1.71-1.86 (m, 6H, CH₂), 4.01-4.06 (m, 6H, ArOCH₂), 7.32 (s, 2H, arom. H).
¹³ C NMR (100.4 MHz, CDCl₃): 5 (ppm) 14.10, 22.685, 25.72, 26.04, 26.07, 29.27, 29.34, 29.36, 29.39, 29.42, 29.55, 29.59, 29.63, 29.65, 29.69, 29.715, 29.73, 29.74, 30.33, 31.92, 31.94, 32.77 (aliphatic), 69.19, 73.55 (ether), 108.56, 123.57, 143.17, 152.85 (aromatic), 171.52 (carbonyl).

### (3) Synthesis of 3,4,5-tris(dodecyloxy)benzyl alcohol

A desired compound (3,4,5-tris(dodecyloxy)benzyl alcohol, OR in the formula [C] is dodecyloxy) (solid, 10.111 g, yield 96.9 %) was obtained similarly as in Example 1 except that 3,4,5-bis(dodecyloxy)benzoate (10.654 g, 15.782 mmol) and LiAlH₄ (0.912 g, 24.029 mmol) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.26-1.36 (m, 48H, CH₂), 1.42-1.495 (m, 6H, CH₂), 1.63 (t, J=6.2 Hz, 1H, OH), 1.70-1.83 (m, 6H, CH₂), 3.91-3.98 (m, 6H, ArOCH₂), 4.59 (d, J=5.6 Hz, 2H, ArCH₂OH), 6.56 (s, 2H, arom. H).
¹³ C NMR (100.4 MHz, CDCl₃): δ (ppm) 14.10, 14.105, 22.685, 22.69, 26.095, 26.14, 29.36, 29.39, 29.41, 29.415, 29.62, 29.64, 29.645, 29.65, 29.69, 29.70, 29.73, 29.75, 30.33, 30.34, 31.92, 31.94 (aliphatic), 65.69, 69.11, 73.425 (ether), 105.37, 136.00, 137.64, 153.29 (aromatic).

### (4) Synthesis of 3,4,5-tris(dodecyloxy)benzaldehyde

A desired compound (3,4,5-tris(dodecyloxy)benzaldehyde, OR in the formula [D] is dodecyloxy) (solid, 8.911 g, yield 88.3 %) was obtained similarly as in Example 1 except that 3,4,5-tris(dodecyloxy)benzyl alcohol (10.126 g, 15.317 mmol), manganese oxide (IV) (6.844 g, 78.724 mmol), and 35 ml of chloroform were used.

¹HNMR and ¹³CNMR of the obtained compound were measured and the following data was obtained.
¹ H NMR (400 MHz, CDCl₃, TMS): δ (ppm) 0.86-0.90 (m,
9H, CH₃), 1.26-1.37 (m, 48H, CH₂), 1.44-1.51 (m, 6H, CH₂), 1.71-1.86 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): δ (ppm) 14.10, 14.11, 22.68, 22.69, 26.02, 26.06, 29.25, 29.35, 29.36, 29.37, 29.38, 29.54, 29.62, 29.65, 29.68, 29.69, 29.71, 29.74, 30.34, 31.915, 31.93 (aliphatic), 69.23, 73.63 (ether), 107.86, 131.435, 143.86, 153.52 (aromatic), 191.27 (aldehyde).

### (5) Synthesis of 5,10,15,20-tetrakis[3,4,5-tris(dodecyloxy)phenyl]porphyrin

A desired compound (5,10,15,20-tetrakis[3,4,5-tris(dodecyloxy)phenyl]porphyrin, OR in the formula [E] is dodecyloxy) (red purple oily material, 0.258 g, yield 9.6 %) was obtained similarly as in Example 1 except that pyrrole (0.35 ml), 3,4,5-tris(dodecyloxy)benzaldehyde (2.516 g, 3.817 mmol), chloroform (400 ml), trifluoroacetic acid(TFA) (0.42 ml), DDQ (0.874 g, 3.850 mmol), triethylamine (1.0 ml), NaBH₄ (0.148 g, 3.912 mmol), and THF-ethanol (10 ml-10 ml (1: 1 v/v)) were used.

¹HNMR and MALDI-TOF-MS of the obtained compound were measured and the following data was obtained.
¹ H NMR (400 MHz, CDCl₃, TMS): δ (ppm) -2.81 (s, 2H, NH), 0.84 (t, J=7.0 Hz, 24H, CH₃), 0.895 (t, J=6.8 Hz, 12H, CH₃), 1.21-1.51 (m, 218H, CH₂), 1.63-1.70 (m, 8H, CH₂), 1.82-1.89 (m, 16H, CH₂), 1.935-2.00 (m, 8H, CH₂), 4.07 (t, J=6.2 Hz, 16H, ArOCH₂), 4.29 (t, J=6.6 Hz, 8H, ArOCH₂), 7.41 (s, 8H, arom. H), 8.94 (s, 8H, pyrrole H).
MALDI-TOF-MS (no matrix): m/z=2827.912 (M⁺).
A photograph of the obtained oily material is shown in FIG. 2.

### (Example 7)

In accordance with the scheme similar to that of Example 1, 5,10,15,20-tetrakis[3,4,5-tris(tridecyloxy)phenyl]porphyrin was produced.

### (1) Synthesis of ethyl 3,4,5-tris(tridecyloxy)benzoate

A desired compound (ethyl 3,4,5-tris(tridecyloxy)benzoate, OR in the formula [A] is tridecyloxy) (solid, 11.778 g, yield 62.3 %) was obtained similarly as in Example 1 except that ethyl 3,4,5-trihydroxybenzoate (5.024 g, 25.351 mmol), potassium carbonate (12.375 g, 89.540 mmol), and 1-bromotridecane (26.0 ml, 95.885 mmol) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.90 (m, 9H, CH₃), 1.26-1.40 (m, 57H, CH₃+CH₂), 1.44-1.51 (m, 6H, CH₂), 1.72-1.85 (m, 6H, CH₂), 3.995-4.03 (m, 6H, ArOCH₂), 4.35 (q, J=7.1 Hz, 2H, CH₃CH₂O), 7.25 (s, 2H, arom. H).
¹³ C NMR (100.4 MHz, CDCl₃): δ (ppm) 14.10, 14.40, 22.685, 25.81, 26.05, 26.08, 28.50, 29.18, 29.31, 29.34, 29.365, 29.38, 29.39, 29.48, 29.54, 29.57, 29.63, 29.66, 29.69, 29.70, 29.73, 29.74, 30.32, 31.92, 31.93 (aliphatic), 60.94, 69.16, 73.47 (ether), 107.975, 125.03, 142.31, 152.79 (aromatic), 166.47 (carbonyl).
MALDI-TOF-MS (matrix: dithranol): m/z=745.68 (M⁺).

### (2) Synthesis of 3,4,5-tris(tridecyloxy)benzoic acid

A desired compound (ethyl 3,4,5-tris(tridecyloxy)benzoate, OR in the formula [B] is tridecyloxy) (solid, 11.128 g, yield 98.4 %) was obtained similarly as in Example 1 except that ethyl 3,4,5-tris(tridecyloxy)benzoate (11.755 g, 15.774 mmol), potassium hydroxide (1.304 g, 23.242 mmol), and THF-methanol-water (80 ml-20 ml-8 ml) were used.

¹HNMR and ¹³CNMR of the obtained compound were measured and the following data was obtained.
¹ H NMR (400 MHz, CDCl₃, TMS): δ (ppm) 0.86-0.90 (m, 9H, CH₃), 1.26-1.35 (m, 54H, CH₂), 1.43-1.49 (m, 6H, CH₂), 1.73-1.86 (m, 6H, CH₂), 4.01-4.06 (m, 6H, ArOCH₂), 7.32 (s, 2H, arom. H).
¹³ C NMR (100.4 MHz, CDCl₃): δ (ppm) 14.10, 14.11, 22.685, 26.04, 26.07, 29.27, 29.34, 29.365, 29.39, 29.42, 29.55, 29.59, 29.60, 29.63, 29.66, 29.69, 29.70, 29.72, 29.74, 30.32, 31.91, 31.92, 31.93, 32.78 (aliphatic), 69.18, 73.54 (ether), 108.53, 123.56, 143.10, 152.84 (aromatic), 171.02 (carbonyl).

### (3) Synthesis of 3,4,5-tris(tridecyloxy)benzyl alcohol

A desired compound (3,4,5-tris(tridecyloxy)benzyl alcohol, OR in the formula [C] is tridecyloxy) (solid, 9.159 g, yield 83.9 %) was obtained similarly as in Example 1 except that 3,4,5-bis(tridecyloxy)benzoic acid (11.128 g, 15.517 mmol), LiAlH₄ (0.909 g, 23.950 mmol), and THF (35 ml) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.90 (m, 9H, CH₃), 1.26-1.36 (m, 54H, CH₂), 1.42-1.48 (m, 6H, CH₂), 1.70-1.81 (m, 6H, CH₂), 3.91-3.985 (m, 6H, ArOCH₂), 4.59 (s, 2H, ArCH₂OH), 6.56 (s, 2H, arom. H).
¹³ C NMR (100.4 MHz, CDCl₃): δ (ppm) 14.13, 14.14, 22.71, 25.75, 26.12, 26.15, 29.37, 29.38, 29.40, 29.43, 29.61, 29.63, 29.64, 29.67, 29.68, 29.72, 29.725, 29.76, 29.77, 30.35, 31.93, 31.94, 31.95, 32.83 (aliphatic), 65.71, 69.13, 73.44 (ether), 105.385, 136.01, 137.66, 153.31 (aromatic).
MALDI-TOF-MS (matrix: dithranol): m/z=703.65 (M⁺), 725.62 ([M+Na]⁺).

### (4) Synthesis of 3,4,5-tris(tridecyloxy)benzaldehyde

A desired compound (3,4,5-tris(tridecyloxy)benzaldehyde, OR in the formula [D] is tridecyloxy) (solid, 6.727 g, yield 74.4 %) was obtained similarly as in Example 1 except that 3,4,5-tris(tridecyloxy)benzyl alcohol (9.068 g, 12.896 mmol) and manganese oxide (IV) (4.831 g, 55.569 mmol) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.90 (m, 9H, CH₃), 1.26-1.37 (m, 54H, CH₂), 1.44-1.51 (m, 6H, CH₂), 1.71-1.86 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): δ (ppm) 14.10, 14.11, 22.68, 22.69, 26.02, 26.06, 29.24, 29.36, 29.37, 29.38, 29.53, 29.54, 29.615, 29.62, 29.65, 29.68, 29.69, 29.71, 29.715, 29.73, 29.735, 30.33, 31.915, 31.93 (aliphatic), 69.22, 73.62 (ether), 107.83, 131.43, 143.83, 153.51 (aromatic), 191.275 (aldehyde). MALDI-TOF-MS (matrix: dithranol): m/z=701.66 (M⁺).

### (5) Synthesis of 5,10,15,20-tetrakis[3,4,5-tris(tridecyloxy)phenyl]porphyrin

A desired compound (5,10,15,20-tetrakis[3,4,5-tris(tridecyloxy)phenyl]porphyrin, OR in the formula [E] is tridecyloxy) (red purple oily material, 0.388 g, yield 14.0 %) was obtained similarly as in Example 1 except that pyrrole (0.30 ml), 3,4,5-tris(tridecyloxy)benzaldehyde (2.591 g, 3.695 mmol), chloroform (390 ml), trifluoroacetic acid (TFA) (0.42 ml), DDQ (0.870 g, 3.832 mmol), triethylamine (1.0 ml), NaBH₄ (0.142 g, 3.753 mmol), and THF-ethanol (10 ml-10 ml (1:1 v/v)) were used.

¹HNMR and MALDI-TOF-MS of the obtained compound were measured and the following data was obtained.
¹ H NMR (400 MHz, CDCl₃, TMS): δ (ppm) -2.81 (s, 2H, NH), 0.84 (t, J=7.0 Hz, 24H, CH₃), 0.89 (t, J=6.6 Hz, 12H, CH₃), 1.21-1.48 (m, 232H, CH₂), 1.63-1.70 (m, 8H, CH₂), 1.82-1.89 (m, 16H, CH₂), 1.93-2.00 (m, 8H, CH₂), 4.07 (t, J=6.2 Hz, 16H, ArOCH₂), 4.29 (t, J=6.4 Hz, 8H, ArOCH₂), 7.41 (s, 8H, arom. H), 8.94 (s, 8H, pyrrole H).
MALDI-TOF-MS (matrix: 2,5-dihydroxybenzoic acid): m/z=2995.86 (M⁺+1).

### (Example 8)

In accordance with the scheme similar to that of Example 1, 5,10,15,20-tetrakis[3,4,5-tris(tetradecyloxy)phenyl]porphyrin was produced.

### (1) Synthesis of ethyl 3,4,5-tris(tetradecyloxy)benzoate

A desired compound (ethyl 3,4,5-tris(tetradecyloxy)benzoate, OR in the formula [A] is tetradecyloxy) (solid, 12.441 g, yield 62.3 %) was obtained similarly as in Example 1 except that ethyl 3,4,5-trihydroxybenzoate (5.028 g, 25.372 mmol), potassium carbonate (12.478 g, 90.286 mmol), and 1-bromotetradecane (25.0 ml, 91.061 mmol) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.895 (m, 9H, CH₃), 1.26-1.40 (m, 63H, CH₃+CH₂), 1.43-1.51 (m, 6H, CH₂), 1.70-1.84 (m, 6H, CH₂), 3.99-4.03 (m, 6H, ArOCH₂), 4.35 (q, J=7.1 Hz, 2H, CH₃CH₂O), 7.25 (s, 2H, arom. H).
¹³ C NMR (100.4 MHz, CDCl₃): δ (ppm) 14.11, 14.12, 14.40, 22.69, 25.81, 26.06, 26.08, 28.50, 29.18, 29.32, 29.35, 29.365, 29.38, 29.40, 29.48, 29.54, 29.57, 29.62, 29.64, 29.665, 29.69, 29.71, 29.74, 29.75, 30.32, 31.915, 31.93 (aliphatic), 60.945, 69.18, 73.475 (ether), 107.98, 125.03, 142.315, 152.79 (aromatic), 166.47 (carbonyl).
MALDI-TOF-MS (matrix: dithranol): m/z=787.67 (M⁺).

### (2) Synthesis of 3,4,5-tris(tetradecyloxy)benzoic acid

A desired compound (ethyl 3,4,5-tris(tetradecyloxy)benzoate, OR in the formula [B] is tetradecyloxy) (solid, 11.711 g, yield 97.9 %) was obtained similarly as in Example 1 except that ethyl 3,4,5-tris(tetradecyloxy)benzoate (12.407 g, 15.759 mmol), potassium hydroxide (1.487 g, 26.504 mmol), and THF-methanol-water (80 ml-20 ml-8 ml) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.90 (m, 9H, CH₃), 1.26-1.35 (m, 60H, CH₂), 1.44-1.51 (m, 6H, CH₂), 1.73-1.86 (m, 6H, CH₂), 4.01-4.06 (m, 6H, ArOCH₂), 7.32 (s, 2H, arom. H).
¹³ C NMR (100.4 MHz, CDCl₃): δ (ppm) 14.11, 14.12, 22.69, 22.695, 25.72, 26.04, 26.07, 26.28, 29.35, 29.37, 29.38, 29.39, 29.43, 29.56, 29.595, 29.61, 29.64, 29.67, 29.69, 29.71, 29.73, 29.74, 29.76, 30.33, 31.93, 32.78 (aliphatic), 69.18, 73.545 (ether), 108.55, 123.545, 143.14, 152.85 (aromatic), 171.27 (carbonyl).
MALDI-TOF-MS (matrix: 2,5-dihydroxybenzoic acid): m/z=759.73 (M⁺), 782.74 ([M+Nal⁺).

### (3) Synthesis of 3,4,5-tris(tetradecyloxy)benzyl alcohol

A desired compound (3,4,5-tris(tetradecyloxy)benzyl alcohol, OR in the formula [C] is tetradecyloxy) (solid, 10.738 g, yield 93.6 %) was obtained similarly as in Example 1 except that 3,4,5-bis(tetradecyloxy)benzoic acid (11.688 g, 15.394 mmol), LiAlH₄ (0.882 g, 23.239 mmol), and THF (35 ml) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.90 (m, 9H, CH₃), 1.26-1.36 (m, 60H, CH₂), 1.42-1.495 (m, 6H, CH₂), 1.70-1.83 (m, 6H, CH₂), 3.91-3.98 (m, 6H, ArOCH₂), 4.59 (s, 2H, ArCH₂OH), 6.56 (s, 2H, arom. H).
¹³ C NMR (100.4 MHz, CDCl₃): δ (ppm) 14.13, 14.135, 22.70, 22.71, 25.75, 26.12, 26.16, 29.37, 29.39, 29.40, 29.44, 29.45, 29.61, 29.63, 29.64, 29.67, 29.69, 29.71, 29.725, 29.77, 29.775, 30.35, 31.95, 31.955, 32.83 (aliphatic), 65.70, 69.13, 73.44 (ether), 105.37, 136.02, 137.63, 153.30 (aromatic).
MALDI-TOF-MS (matrix: dithranol): m/z=745.79 (M⁺), 768.78 ([M+Na]⁺).

### (4) Synthesis of 3,4,5-tris(tetradecyloxy)benzaldehyde

A desired compound (3,4,5-tris(tetradecyloxy)benzaldehyde, OR in the formula [D] is tetradecyloxy) (solid, 8.401 g, yield 79.0 %) was obtained similarly as in Example 1 except that 3,4,5-tris(tetradecyloxy)benzyl alcohol (10.662 g, 14.306 mmol) and manganese oxide (IV) (10.264 g, 0.118 mol) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.895 (m, 9H, CH₃), 1.26-1.37 (m, 60H, CH₂), 1.44-1.51 (m, 6H, CH₂), 1.71-1.86 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): δ (ppm) 14.10, 14.13, 22.685, 22.71, 26.02, 26.06, 29.245, 29.25, 29.36, 29.37, 29.535, 29.54, 29.56, 29.62, 29.625, 29.65, 29.69, 29.70, 29.705, 29.715, 29.72, 29.735, 29.74, 30.34, 31.92, 31.93 (aliphatic), 69.23, 73.62 (ether), 107.84, 131.43, 143.85, 153.51 (aromatic), 191.28 (aldehyde).
MALDI-TOF-MS (matrix: 2,5-dihydroxybenzoic acid): m/z=743.81 (M⁺), 766.79([M+Na]⁺).

### (5) Synthesis of 5,10,15,20-tetrakis[3,4,5-tris(tetradecyloxy)phenyl]porphyrin

A desired compound (5,10,15,20-tetrakis[3,4,5-tris(tetradecyloxy)phenyl]porphyrin, OR in the formula [E] is tetradecyloxy) (red purple oily material, 0.450 g, yield 12.0 %) was obtained similarly as in Example 1 except that pyrrole (0.35 ml), 3,4,5-tris(tetradecyloxy)benzaldehyde (3.510 g, 4.722 mmol), chloroform (500 ml), trifluoroacetic acid (TFA) (0.54 ml), DDQ (1.176 g, 5.180 mmol), triethylamine (1.0 ml), NaBH₄ (0.172 g, 4.546 mmol), and THF-ethanol (10 ml-10 ml (1: 1 v/v)) were used.

¹HNMR and MALDI-TOF-MS of the obtained compound were measured and the following data was obtained.
¹ H NMR (400 MHz, CDCl₃, TMS): δ (ppm) -2.81 (s, 2H, NH), 0.845 (t, J=6.8 Hz, 24H, CH₃), 0.88 (t, J=6.8 Hz, 12H, CH₃), 1.21-1.52 (m, 256H, CH₂), 1.63-1.70 (m, 8H, CH₂), 1.80-1.89 (m, 16H, CH₂), 1.93-2.00 (m, 8H, CH₂), 4.07 (t, J=6.4 Hz, 16H, ArOCH₂), 4.29 (t, J=6.8 Hz, 8H, ArOCH₂), 7.41 (s, 8H, arom. H), 8.93 (s, 8H, pyrrole H).
MALDI-TOF-MS (matrix: 2,5-dihydroxybenzoic acid): m/z=3164.11 (M⁺+1).

### (Comparative example 1)

(5,10,15,20-tetrakis[3,4,5-tris(pentyloxy)phenyl]porphyrin) was produced similarly as in Example 1 except that the formula [C] was synthesized from the formula [A] without going through the formula [B] as shown in the following scheme.

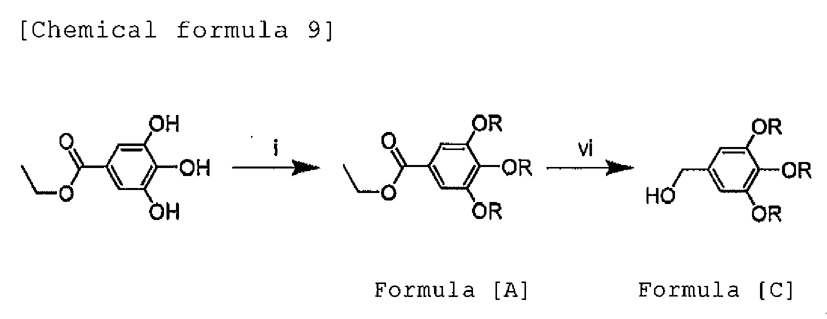

i) alkylbromide, K₂CO₃, DMF, 90 degree. vi) LiAlH₄, THF, reflux.

### (1) Synthesis of ethyl 3,4,5-tris(pentyloxy)benzoate

A desired compound (ethyl 3,4,5-tris(pentyloxy)benzoate, OR in the formula [A] is pentyloxy) (oily material, 7.014 g, yield 67.8 %) was obtained similarly as in Example 1 except that ethyl 3,4,5-trihydroxybenzoate (5.017 g, 25.316 mmol), potassium carbonate (12.268 g, 88.766 mmol), and 1-bromopentane (12.5 ml, 0.101 mol) were used.

¹HNMR and ¹³CNMR of the obtained compound were measured and the following data was obtained.
¹ H NMR (400 MHz, CDCl₃, TMS): δ (ppm) 0.90-0.95 (m, 9H, CH₃), 1.32-1.51 (m, 15H, CH₃+CH₂), 1.72-1.86 (m, 6H, CH₂), 4.00-4.03 (m, 6H, ArOCH₂), 4.35 (q, J=7.2 Hz, 2H, CH₃CH₂O), 7.255 (s, 2H, arom. H).
¹³ C NMR (100.4 MHz, CDCl₃): δ (ppm) 14.005, 14.05, 14.39, 22.41, 22.52, 28.17, 28.23, 28.98, 29.96 (aliphatic), 60.94, 69.17, 73.43 (ether), 108.01, 125.055, 142.33, 152.80 (aromatic), 166.46 (carbonyl).

### (2) Synthesis of 3,4,5-tris(pentyloxy)benzyl alcohol

Ethyl 3,4,5-bis(pentyloxy)benzoate (7.003 g, 17.140 mmol) and lithium aluminum hydride (LiAlH₄) (0.703 g, 18.522 mmol) were refluxed in 50 ml of THF under an argon atmosphere overnight. After cooling the mixture to room temperature, the mixture was agitated while being cooled with ice, and ethyl acetate was added therein to deactivate the remaining LiAlH₄ followed by adding a small amount of water and further agitating the mixture. Next, chloroform was added therein and agitated at room temperature, and anhydrous magnesium sulfate was further added therein and agitated for several tens of minutes. After the solution was filtrated, the filtrate was condensed. Thus obtained residue was purified by Silica Gel Column Chromatography (developing solvent: chloroform → chloroform-methanol (98: 2 v/v)). Thus, a desired compound (3,4,5-tris(pentyloxy)benzyl alcohol, OR in the formula [C] is pentyloxy) (solid, 4.155 g, yield 66.1 %) was obtained.

¹HNMR and ¹³CNMR of the obtained compound were measured and the following data was obtained.
¹ H NMR (400 MHz, CDCl₃, TMS): δ (ppm) 0.92 (t, J=7.2 Hz, 3H, CH₃), 0.925 (t, J=7.4 Hz, 6H, CH₃), 1.32-1.50 (m, 12H, CH₂), 1.65 (br, 1H, OH), 1.71-1.84 (m, 6H, CH₂), 3.94 (t, J=7.0 Hz, 2H, ArOCH₂), 3.97 (t, J=6.6 Hz, 4H, ArOCH₂), 4.59 (s, 2H, ArCH₂O), 6.56 (s, 2H, arom. H).
¹³ C NMR (100.4 MHz, CDCl₃): δ (ppm) 14.02, 14.07, 22.43, 22.56, 22.59, 28.24, 29.085, 29.96 (aliphatic), 65.67, 69.11, 73.37 (ether), 105.37, 136.02, 137.62, 153.29 (aromatic).

### (3) Synthesis of 3,4,5-tris(pentyloxy)benzaldehyde

A desired compound (3,4,5-tris(pentyloxy)benzaldehyde, OR in the formula [D] is pentyloxy) (oily material, 3.460 g, yield 83.7 %) was obtained similarly as in Example 1 except that 3,4,5-tris(pentyloxy)benzyl alcohol (4.145 g, 11.336 mmol), manganese oxide (IV) (4.026 g, 46.309 mmol), and 35 ml of chloroform were used.

¹HNMR and ¹³CNMR of the obtained compound were measured and the following data was obtained.
¹ H NMR (400 MHz, CDCl₃, TMS): δ (ppm) 0.93 (t, J=7.0 Hz, 3H, CH₃), 0.94 (t, J=7.0 Hz, 6H, CH₃), 1.33-1.51 (m, 12H, CH₂), 1.73-1.875 (m, 6H, CH₂), 4.04 (t, J=6.6 Hz, 4H, ArOCH₂), 4.06 (t, J=6.6 Hz, 2H, ArOCH₂), 7.085 (s, 2H, arom. H), 9.83 (s, 1H, CHO).
¹³ C NMR (100.4 MHz, CDCl₃): δ(ppm) 14.00, 14.05, 22.40, 22.49, 28.13, 28.20, 28.91, 29.97 (aliphatic), 69.21, 73.57 (ether), 107.83, 131.435, 143.82, 153.51 (aromatic), 191.29 (aldehyde).

### (4) Synthesis of 5,10,15,20-tetrakis[3,4,5-tris(pentyloxy)phenyl]porphyrin

A desired compound (5,10,15,20-tetrakis[3,4,5-tris(pentyloxy)phenyl]porphyrin, OR in the formula [E] is pentyloxy) (red purple solid, 0.307 g, yield 13.5 %) was obtained similarly as in Example 1 except that pyrrole (0.40 ml), 3,4,5-tris(pentyloxy)benzaldehyde (2.017 g, 5.533 mmol), chloroform (550 ml), trifluoroacetic acid (TFA) (0.62 ml), DDQ (1.251 g, 5.511 mmol), triethylamine (1.5 ml), NaBH₄ (0.220 g, 5.815 mmol), and THF-ethanol (10 ml-10 ml (1: 1 v/v)) were used.

¹HNMR and MALDI-TOF-MS of the obtained compound were measured and the following data was obtained. ¹ H NMR (400 MHz, CDCl₃, TMS): δ (ppm) -2.81 (s, 2H, NH), 0.90 (t, J=7.4 Hz, 24H, CH₃), 1.03 (t, J=7.2 Hz, 12H, CH₃), 1.33-1.55 (m, 40H, CH₂), 1.62-1.70 (m, 8H, CH₂), 1.84-1.91 (m, 16H, CH₂), 1.95-2.02 (m, 8H, CH₂), 4.085 (t, J=6.2 Hz, 16H, ArOCH₂), 4.30 (t, J=6.6 Hz, 8H, ArOCH₂), 7.42 (s, 8H, arom. H), 8.945 (s, 8H, pyrrole H).
MALDI-TOF-MS (no matrix): m/z=1649.56 (M⁺+1).

### (Comparative example 2)

(5,10,15,20-tetrakis[3,4,5-tris(hexyloxy)phenyl]porphyrin) was produced similarly as in Comparative example 1.

### (1) Synthesis of ethyl 3,4,5-tris(hexyloxy)benzoate

A desired compound (ethyl 3,4,5-tris(hexyloxy)benzoate, OR in the formula [A] is hexyloxy) (oily material, 9.377 g, yield 81.7 %) was obtained similarly as in Example 1 except that ethyl 3,4,5-trihydroxybenzoate (5.047 g, 25.467 mmol), potassium carbonate (12.287 g, 88.904 mmol), and 1-bromohexane (15.0 ml, 0.106 mol) were used.

¹HNMR and ¹³CNMR of the obtained compound were measured and the following data was obtained.
¹ H NMR (400 MHz, CDCl₃, TMS): δ (ppm) 0.88-0.925 (m, 9H, CH₃), 1.29-1.36 (m, 12H, CH₂), 1.38 (t, J=7.0 Hz, 2H, CH₃CH₂O), 1.45-1.52 (m, 6H, CH₂), 1.71-1.85 (m, 6H, CH₂), 4.00-4.03 (m, 6H, ArOCH₂), 4.35 (q, J=7.2 Hz, 2H, CH₃CH₂O), 7.255 (s, 2H, arom. H).
¹³ C NMR (100.4 MHz, CDCl₃): δ (ppm) 13.99, 14.05, 14.39, 22.60, 22.65, 25.69, 25.73, 29.27, 30.26, 31.545, 31.72 (aliphatic), 60.94, 69.21, 73.98 (ether), 108.06, 125.06, 142.38, 152.805 (aromatic), 166.97 (carbonyl).

### (2) Synthesis of 3,4,5-tris(hexyloxy)benzyl alcohol

A desired compound (3,4,5-tris(hexyloxy)benzyl alcohol, OR in the formula [C] is hexyloxy) (solid, 6.201 g, yield 73.0 %) was obtained similarly as in Comparative example 1 except that ethyl 3,4,5-bis(hexyloxy)benzoate (9.366 g, 20.783 mmol), lithium aluminum hydride (LiAlH₄) (1.188 g, 31.301 mmol), and 35 ml of THF were used.

¹HNMR and ¹³CNMR of the obtained compound were measured and the following data was obtained.
¹ H NMR (400 MHz, CDCl₃, TMS): δ (ppm) 0.88-0.92 (m, 9H, CH₃), 1.29-1.37 (m, 12H, CH₂), 1.43-1.51 (m, 12H, CH₂), 1.65 (t, J=5.8 Hz, 1H, OH), 1.70-1.83 (m, 6H, CH₂), 3.92-3.99 (m, 6H, ArOCH₂), 4.59 (d, J=6.0 Hz, 2H, ArCH₂O), 6.59 (s, 2H, arom. H).
¹³ C NMR (100.4 MHz, CDCl₃): δ (ppm) 14.02, 14.08, 22.615, 22.68, 25.745, 25.76, 29.35, 30.25, 31.56, 31.77 (aliphatic), 65.675, 69.08, 73.41 (ether), 105.31, 136.005, 137.56, 153.265 (aromatic).

### (3) Synthesis of 3,4,5-tris(hexyloxy)benzaldehyde

A desired compound (3,4,5-tris(hexyloxy)benzaldehyde, OR in the formula [D] is hexyloxy) (oily material, 5.343 g, yield 87.5 %) was obtained similarly as in Example 1 except that 3,4,5-trishexyloxybenzyl alcohol (6.134 g, 15.011 mmol) and manganese oxide (IV) (5.323 g, 61.228 mmol) were used.

¹HNMR and ¹³CNMR of the obtained compound were measured and the following data was obtained.
¹ H NMR (400 MHz, CDCl₃, TMS): δ (ppm) 0.885-0.93 (m, 9H, CH₃), 1.30-1.38 (m, 12H, CH₂), 1.45-1.52 (m, 6H, CH₂), 1.72-1.87 (m, 6H, CH₂), 4.04 (t, J=6.6 Hz, 4H, ArOCH₂), 4.06 (t, J=6.6 Hz, 2H, ArOCH₂), 7.085 (s, 2H, arom. H), 9.83 (s, 1H, CHO).
¹³ C NMR (100.4 MHz, CDCl₃): δ (ppm) 14.005, 14.06, 22.59, 22.65, 25.65, 25.71, 29.17, 30.26, 31.51, 31.68 (aliphatic), 69.18, 73.60 (ether), 107.765, 131.41, 143.76, 153.49 (aromatic), 191.32 (aldehyde).

### (4) Synthesis of 5,10,15,20-tetrakis[3,4,5-tris(hexyloxy)phenyl]porphyrin

A desired compound (5,10,15,20-tetrakis[3,4,5-tris(hexyloxy)phenyl]porphyrin, OR in the formula [E] is hexyloxy) (red purple oily material, 0.241 g, yield 10.4 %) was obtained similarly as in Example 1 except that pyrrole (0.40 ml), 3,4,5-tris(hexyloxy)benzaldehyde (2.083 g, 5.123 mmol), chloroform (500 ml), trifluoroacetic acid (TFA) (0.6 ml), DDQ (1.220 g, 5.374 mmol), triethylamine (1.5 ml), NaBH₄ (0.208 g, 5.498 mmol), and THF-ethanol (10 ml-10 ml (1: 1 v/v)) were used. The desired compound was an oily material right after the preparation. However, several months later, the oily material partially solidified.

¹HNMR and MALDI-TOF-MS of the obtained compound were measured and the following data was obtained.
¹ H NMR (400 MHz, CDCl₃, TMS): δ (ppm) -2.82 (s, 2H, NH), 0.86 (t, J=7.2 Hz, 24H, CH₃), 0.99 (t, J=6.8 Hz, 12H, CH₃), 1.245-1.35 (m, 32H, CH₂), 1.42-1.54 (m, 32H, CH₂), 1.63-1.71 (m, 8H, CH₂), 1.83-1.90 (m, 16H, CH₂), 1.935-2.005 (m, 8H, CH₂), 4.08 (t, J=6.2 Hz, 16H, ArOCH₂), 4.30 (t, J=6.6 Hz, 8H, ArOCH₂), 7.42 (s, 8H, arom. H), 8.945 (s, 8H, pyrrole H).
MALDI-TOF-MS (no matrix): m/z=1816.17 (M⁺).

### (Example 9)

Similarly as in Comparative example 1, (5,10,15,20-tetrakis[3,4,5-tris(pentadecyloxy)phenyl]porphyrin) was produced by synthesizing the formula [C] from the formula [A] without going through the formula [B].

### (1) Synthesis of ethyl 3,4,5-tris(pentadecyloxy)benzoate

Ethyl 3,4,5-trihydroxybenzoate (5.003 g, 25.245 mmol) and potassium carbonate (12.245 g, 88.600 mmol) were agitated in DMF (35 ml) in the presence of 1-bromopentadecane (25.5 ml, 87.972 mmol) under an argon atmosphere at 90 °C. After the reacted product was extracted into an organic phase using chloroform-water, the organic phase was washed twice by water, further washed by saturated sodium thiosulfate solution, and dried by anhydrous magnesium sulfate. After the organic phase was filtrated, the filtrate was condensed. Thus obtained residue was recrystalized twice by dichloromethane-methanol, thus, a desired compound (ethyl 3,4,5-tris(pentadecyloxy)benzoate, OR in the formula [A] is pentadecyloxy) (white powder, 18.758 g, yield 89.6 %) was obtained.

¹HNMR, ¹³CNMR and MALDI-TOF-MS of the obtained compound were measured and the following data was obtained.
¹ H NMR (400 MHz, CDCl₃, TMS): δ(ppm) 0.86-0.895 (m, 9H, CH₃), 1.26-1.40 (m, 69H, CH₃+CH₂), 1.43-1.51 (m,
6H, CH₂), 1.70-1.84 (m, 6H, CH₂), 3.99-4.03 (m, 6H, ArOCH₂), 4.35 (q, J=7.1 Hz, 2H, CH₃CH₂O), 7.25 (s, 2H, arom. H).
¹³ C NMR (100.4 MHz, CDCl₃): δ(ppm) 14.10, 14.12, 14.40, 22.685, 22.705, 26.06, 26.08, 29.315, 29.32, 29.36, 29.365, 29.37, 29.40, 29.565, 29.57, 29.63, 29.64, 29.66, 29.68, 29.70, 29.705, 29.71, 29.73, 29.74, 29.75, 30.32, 30.325, 31.92, 31.93 (aliphatic), 60.94, 69.18, 73.475 (ether), 108.00, 125.03, 142.33, 152.79 (aromatic), 166.47 (carbonyl).
MALDI-TOF-MS (matrix: 2,5-dihydroxybenzoic acid): m/z=829.01 (M⁺), 852.03 ([M+Na]⁺)

### (2) Synthesis of 3,4,5-tris(pentadecyloxy)benzyl alcohol

Ethyl 3,4,5-tris(pentadecyloxy)benzoate (10.072 g, 12.144 mmol) and lithium aluminum hydride (LiAlH₄; LAH) (0.728 g, 19.181 mmol) were refluxed in THF (50 ml) under an argon atmosphere overnight. After cooling the mixture to room temperature, the mixture was agitated while being cooled with ice, and ethyl acetate was added therein to deactivate the remaining LAH followed by adding a small amount of water and further agitating the mixture. Next, chloroform was added therein and agitated at room temperature, and anhydrous magnesium sulfate was further added therein and agitated for several tens of minutes. After the solution was filtrated, the filtrate was condensed. Thus obtained residue was recrystalized by dichloromethane-methanol, thus, a desired compound (3,4,5-tris(pentadecyloxy)benzyl alcohol, OR in the formula [C] is pentadecyloxy) (white powder, 8.659 g, yield 90.6 %) was obtained.

¹HNMR, ¹³CNMR and MALDI-TOF-MS of the obtained compound were measured and the following data was obtained.
¹ H NMR (400 MHz, CDCl₃, TMS): δ(ppm) 0.86-0.90 (m, 9H, CH₃), 1.26-1.36 (m, 66H, CH₂), 1.92-1.50 (m, 6H, CH₂), 1.62 (t, J=5.8 Hz, 1H, OH), 1.70-1.83 (m, 6H, CH₂), 3.91-3.98 (m, 6H, ArOCH₂), 4.59 (d, J=6.4 Hz, 2H, ArCH₂O), 6.56 (s, 2H, arom. H).
¹³ C NMR (100.4 MHz, CDCl₃): δ(ppm) 14.10, 14.11, 22.69, 26.10, 26.14, 29.365, 29.38, 29.42, 29.62, 29.625, 29.65, 29.655, 29.665, 29.68, 29.71, 29.74, 29.75, 29.76, 30.33, 31.93 (aliphatic), 65.69, 69.11, 73.425 (ether), 105.37, 136.00, 137.63, 153.29 (aromatic).
MALDI-TOF-MS (matrix:2,5-dihydroxybenzoic acid): m/z=787.05 (M⁺), 810.06 ([M+Na]⁺).

### (3) Synthesis of 3,4,5-tris(pentadecyloxy)benzaldehyde

A desired compound (3,4,5-tris(pentadecyloxy)benzaldehyde, OR in the formula [D] is pentadecyloxy) (white powder, 6.091 g, yield 85.6 %) was obtained similarly as in Example 1 except that 3,4,5-trispentadecyloxybenzyl alcohol (7.135 g, 9.062 mmol) and manganese oxide (IV) (4.160 g, 47.851 mmol) were used.

¹HNMR, ¹³CNMR and MALDI-TOF-MS of the obtained compound were measured and the following data was obtained.
¹ H NMR (400 MHz, CDCl₃, TMS): δ (ppm) 0.86-0.90 (m, 9H, CH₃), 1.26-1.37 (m, 66H, CH₂), 1.44-1.51 (m, 6H, CH₂), 1.71-1.86 (m, 6H, CH₂), 4.02-4.07 (m, 6H, ArOCH₂), 7.08 (s, 2H, arom. H), 9.83 (s, 1H, CHO).
¹³ C NMR (100.4 MHz, CDCl₃): δ (ppm) 14.11, 14.12, 22.69, 26.025, 26.07, 29.25, 29.365, 29.38, 29.55, 29.63, 29.665, 29.68, 29.69, 29.71, 29.74, 30.34, 31.93 (aliphatic), 69.23, 73.63 (ether), 107.84, 131.43, 143.85, 153.52 (aromatic), 191.29 (aldehyde).
MALDI-TOF-MS (matrix: 2,5-dihydroxybenzoic acid): m/z=785.92 (M⁺), 808.91 ([M+Na]⁺).

### (4) Synthesis of 5,10,15,20-tetrakis[3,4,5-tris(pentadecyloxy)phenyl]porphyrin

A mixture of pyrrole (0.40 ml) and 3,4,5-tris(pentadecyloxy)benzaldehyde (3.700 g, 4.711 mmol) was agitated in chloroform (500 ml) at room temperature, and argon was bubbled in the solution for an hour. Subsequently, trifluoroacetic acid (TFA) (0.52 ml) was added therein and agitated at room temperature overnight. To thus obtained solution, 2,3-dichloro-5,6-dicyanobenzoquinone (DDQ) (1.075 g, 4.736 mmol) was added, and further agitated for 5 hours. Next, triethylamine (1.2 ml) was added therein and the solution was hydrolyzed followed by being condensed. Then, the obtained residue was passed through silica gel using chloroform as a developing solvent followed by being purified by Silica Gel Column Chromatography (developing solvent: chloroform-hexane (2: 1 v/v)) and freeze-dried. Thus, a desired compound (5,10,15,20-tetrakis[3,4,5-tris(pentadecyloxy)phenyl]porphyrin, OR in the formula [E] is pentadecyloxy) (red purple solid, 0.186 g, yield 4.7 %) was obtained. Since there was a difference of polarity between the unreacted material and the desired compound, the step of purifying the desired compound by converting a benzaldehyde derivative to a benzyl alcohol derivative was not required.

¹HNMR and MALDI-TOF-MS of the obtained compound were measured and the following data was obtained.
¹ H NMR (400 MHz, CDCl₃, TMS): δ(ppm) -2.81 (s, 2H, NH), 0.85 (t, J=6.8 Hz, 24H, CH₃), 0.88 (t, J=6.4 Hz, 12H, CH₃), 1.21-1.52 (m, 280H, CH₂), 1.63-1.70 (m, 8H, CH₂), 1.825-1.89 (m, 16H, CH₂), 1.935-2.005 (m, 8H, CH₂), 4.08 (t, J=6.6 Hz, 16H, ArOCH₂), 4.29 (t, J=6.6 Hz, 8H, ArOCH₂), 7.415 (s, 8H, arom. H), 8.94 (s, 8H, pyrrole H).
MALDI-TOF-MS (matrix:2,5-dihydroxybenzoic acid): m/z=3331.39 (M⁺).

The thermal properties of the above-obtained porphyrin derivatives were evaluated as follows.
Thermogravimetry/Differential Thermal Analysis (TG-DTA) was performed at temperatures from 30 to 600 °C using simultaneous TG/DTA instrument (product name: DTG-60A; manufactured by SHIMADZU CORPORATION) under a nitrogen atmosphere at a heating rate of 10 K/min, and a 2 % weight loss temperature was defined as a thermal decomposition temperature. In addition, a melting point (Tm) and a freezing point (Tf) were determined by performing a differential scanning calorimetry (DSC) at temperatures from -100 to 100 °C using Differential Scanning Calorimeter (product name: DSC-60; manufactured by SHIMADZU CORPORATION) under a nitrogen atmosphere at a heating and cooling rate of 10 K/min. In the measurement of the melting point (Tm) and freezing point (Tf), heating and cooling were performed twice respectively. The results are shown in Table 1.

Table 1.

| | Substituent OR | Number of carbon atoms | Td | Tm(1st) | Tf(1st) | Tm(2nd) | Tf (2nd) |
|---|---|---|---|---|---|---|---|
| Example 1 | n-heptyloxy | 7 | 292.81 | ND | ND | ND | ND |
| Example 2 | n-octyloxy | 8 | 353.71 | ND | ND | ND | ND |
| Example 3 | n-nonyloxy | 9 | 229.17 | ND | ND | ND | ND |
| Example 4 | n-decyloxy | 10 | 255.12 | -39.36 | vw | -40.77 | vw |
| Example 5 | n-undecyloxy | 11 | 300.93 | -23.02 | -35.34 | -23.48 | -35.41 |
| Example 6 | n-dodecyloxy | 12 | 230.42 | -5.96 | -20.94 | -5.87 | -21.02 |
| Example 7 | n-tridecyloxy | 13 | 283.67 | 11.15 | -3.31 | 11 | -3.35 |
| Example 8 | n-tetradecyloxy | 14 | 323.27 | 18.44 | 6.4 | 18.59 | 6.52 |
| Example 9 | n-pentadecyloxy | 15 | 288.24 | 34.26 | 16.96 | 27.56 | 17.08 |
| Comparative example 1 | n-pentyloxy | 5 | 333.99 | 78.62 | ND | ND | ND |
| Comparative example 2 | n-hexyloxy | 6 | 250.63 | 83.88 | ND | 83.92 | ND |

| | | | | | | | |
|---|---|---|---|---|---|---|---|
| Td: Decomposition temperature (2 wt%)/°C Tm: Melting point (1st + 2nd scan)/°C Tf: Freezing point (1st +2nd scan)/°C ND: Not determined or Not observed. vw: very weak | | | | | | | |

From the results of Table 1, it can be suggested that the thermal property was dependent on the types of the substituent. The porphyrin derivatives of Examples 4 to 6, in which three alkoxy groups having linear alkyl groups having 10 to 12 carbon atoms were substituted, were in the liquid state at less than 0 °C. In the porphyrin derivatives of Examples 1 to 3 having linear alkyl groups having 7 to 9 carbon atoms, the peak of the melting point by DTA was not observed within the range from 30 to 600 °C by measurement of TG-DTA, and further, the melting point or the freezing point thereof was not observed within the range from - 100 to 100 °C by measurement of DSC-60. The porphyrin derivatives of Examples 1 to 3 may possibly have the melting point or the freezing point at -100 °C or less.
The porphyrin derivative of Example 9, in which the alkoxy group having 15 carbon atoms was substituted, was not able to attain the liquid state at 25 °C since the melting point (Tm) was over 25 °C, however, the porphyrin derivative of Example 9 was in the liquid state at temperatures from 26 to 40 °C. In the porphyrin derivative of Example 9, in which the alkoxy group having 15 carbon atoms was substituted, compared with other derivatives having the alkoxy group having 14 or less carbon atoms, the melting point obtained in the first scan was different from that obtained in the second scan, however, the difference between the melting points obtained in the second and third scan was within the range of measurement error. It can be considered that the higher melting point was observed in the first scan since molecules were aggregated if the porphyrin derivative was in the solid state, however, the substance-specific melting point was observed since the aggregation of molecules was inhibited if the porphyrin derivative was melted once.
The porphyrin derivative of Comparative example 2, in which the alkoxy group having 6 carbon atoms was substituted, was oily material right after the preparation, however, it partially solidified several months later, and the peak was observed around 80 °C by the measurement of DSC. The reason thereof can be considered that molecules partially aggregate. The porphyrin derivative of Comparative example 2 may possibly have two phases including a solid state and liquid state.
Considering the relationship of the numbers of carbon atoms of the substituents OR which are the same kinds at the same substitution position, from the results of Examples and Comparative examples, it can be suggested that the porphyrin derivative cannot attain the liquid state at 25 °C or at temperatures from 26 to 40 °C if the number of carbon atoms is too small or too large.

## Claims

1. A liquid porphyrin derivative at 25 °C represented by the following formula (1): 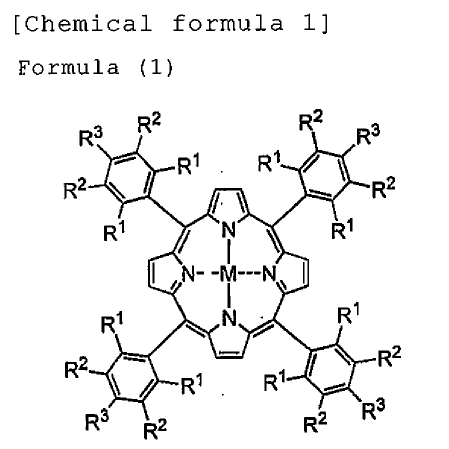 wherein M represents 2H (hydrogen atoms) or an atom or compound capable of binding covalently or coordinately to tetraphenylporphyrin; each of R¹, R², and R³ independently represents a hydrogen atom, or an alkoxy group having 7 to 14 carbon atoms represented by OR⁴ ; R⁴ represents a substituted or unsubstituted alkyl group having 7 to 14 carbon atoms; all the R¹s, all the R²s, and all the R³s are respectively the same; and there are cases where the R²s and the R³s are the alkoxy groups having 7 to 14 carbon atoms represented by OR⁴, and the R¹s are hydrogen atoms, where the R¹s and the R³s are the alkoxy groups having 7 to 14 carbon atoms represented by OR⁴ and the R²s are hydrogen atoms, where the R¹s and the R²s are the alkoxy groups having 7 to 14 carbon atoms represented by OR⁴, and the R³s are hydrogen atoms, and where the R¹s, the R²s and the R³s are the alkoxy groups having 7 to 14 carbon atoms represented by OR⁴.

2. The liquid porphyrin derivative according to Claim 1, wherein, in the formula (1), the R²s and the R³s are the alkoxy groups having 7 to 14 carbon atoms represented by OR⁴, and the R¹s are hydrogen atoms.

3. The liquid porphyrin derivative according to Claim 1 or 2, wherein, in the formula (1), R⁴ is a substituted or unsubstituted linear alkyl group.

4. A method for producing a liquid porphyrin derivative represented by the following formula (1): 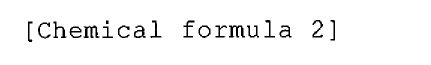 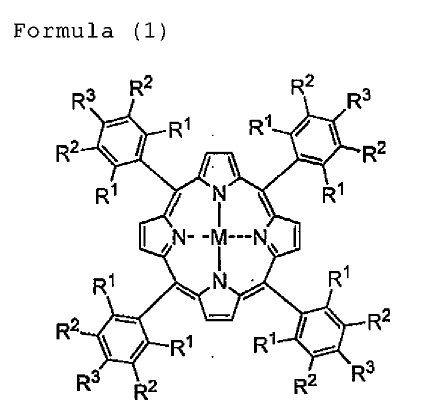 wherein M represents 2H (hydrogen atoms) or an atom or compound capable of binding covalently or coordinately to tetraphenylporphyrin; each of R¹, R², and R³ independently represents a hydrogen atom, or an alkoxy group having 7 to 14 carbon atoms represented by OR⁴; R⁴ represents a substituted or unsubstituted alkyl group having 7 to 14 carbon atoms; all the R¹s, all the R²s, and all the R³s are respectively the same; and there are cases where the R²s and the R³s are the alkoxy groups having 7 to 14 carbon atoms represented by OR⁴, and the R¹s are hydrogen atoms, where the R¹s and the R³s are the alkoxy groups having 7 to 14 carbon atoms represented by OR⁴, and the R²s are hydrogen atoms, where the R¹s and R²s are the alkoxy group having 7 to 14 carbon atoms represented by OR⁴, and the R³s are hydrogen atoms, and where the R¹s, the R²s and the R³s are the alkoxy groups having 7 to 14 carbon atoms represented by OR⁴,
comprising the steps of:
reacting a benzaldehyde derivative with pyrrole, and
purifying the porphyrin derivative after reducing the unreacted benzaldehyde derivative to a benzyl alcohol derivative.

5. A liquid porphyrin derivative at temperatures from 26 to 40 °C represented by the following formula (1): 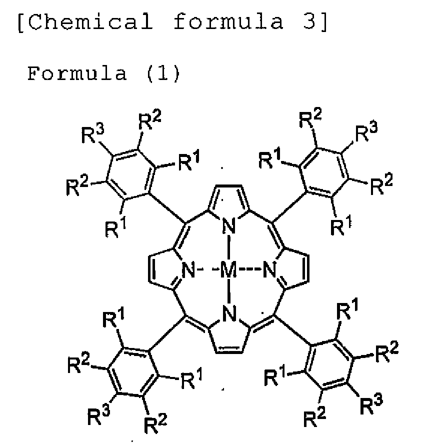 wherein M represents 2H (hydrogen atoms) or an atom or compound capable of binding covalently or coordinately to tetraphenylporphyrin; each of R¹, R², and R³ independently represents a hydrogen atom, or an alkoxy group having 15 carbon atoms represented by OR⁴; R⁴ represents a substituted or unsubstituted alkyl group having 15 carbon atoms; and all the R¹s, all the R²s, and all the R³s are respectively the same; and there are cases where the R²s and the R³s are the alkoxy groups having 15 carbon atoms represented by OR⁴, and the R¹s are hydrogen atoms, where the R¹s and the R³s are the alkoxy groups having 15 carbon atoms represented by OR⁴, and the R²s are hydrogen atoms, where the R¹s and t h e R²s are the alkoxy groups having 15 carbon atoms represented by OR⁴, and the R³s are hydrogen atoms, and where the R¹s, the R²s and the R³s are the alkoxy groups having 15 carbon atoms represented by OR⁴.
